(19) Europäisches Patentamt
European Patent Office
Office européen des brevets

(11) Veröffentlichungsnummer: **0 361 069 B1**

# (12) EUROPÄISCHE PATENTSCHRIFT

(45) Veröffentlichungstag der Patentschrift: **15.06.94**

(51) Int. Cl.$^5$: **C07D 235/24**, C08K 5/3447, C07D 405/06

(21) Anmeldenummer: **89115338.9**

(22) Anmeldetag: **19.08.89**

Die Akte enthält technische Angaben, die nach dem Eingang der Anmeldung eingereicht wurden und die nicht in dieser Patentschrift enthalten sind.

## (54) Benzimidazol-2-carbonsäureanilide, ihre Verwendung als Lichtschutzmittel für organisches Material und mit diesen Aniliden stabilisiertes organisches Material.

(30) Priorität: **23.08.88 DE 3828535**

(43) Veröffentlichungstag der Anmeldung:
**04.04.90 Patentblatt 90/14**

(45) Bekanntmachung des Hinweises auf die Patenterteilung:
**15.06.94 Patentblatt 94/24**

(84) Benannte Vertragsstaaten:
**BE CH DE ES FR GB IT LI NL**

(56) Entgegenhaltungen:
**EP-A- 0 269 230**
**EP-A- 0 284 828**
**US-A- 3 907 700**
**US-A- 4 011 236**

(73) Patentinhaber: **BASF Aktiengesellschaft**
**Carl-Bosch-Strasse 38**
**D-67063 Ludwigshafen(DE)**

(72) Erfinder: **Spang, Peter, Dr.**
**Zur Audell 43**
**D-6670 St. Ingbert(DE)**
Erfinder: **Neumann, Peter, Dr.**
**Poststrasse 28**
**D-6800 Mannheim 1(DE)**
Erfinder: **Trauth, Hubert**
**Milanstrasse 5**
**D-6724 Dudenhofen(DE)**

EP 0 361 069 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus den US-PS 3 907 700 und 4 011 236 sind bereits Lichtschutzmittel für Kunststoffe auf Benzimidazol-Basis bekannt. Es handelt sich dabei um N-(Benzimidazol-2-yl)-phenylcarboxamide der Formel (X)

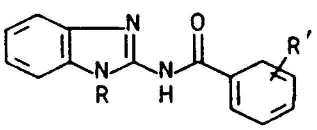

(X)

Diese Stabilisatoren sind zum Teil nur nach aufwendigen verfahren herstellbar. Außerdem zeigen sie in besonders strahlungsempfindlichen Kunststoffen, wie z.B. Polyurethanen, eine nicht ausreichende Lichtschutzwirkung. Weiterhin sind diese Verbindungen größtenteils schwer löslich und relativ flüchtig. Sie neigen zur Kristallisation und Migration. Diese ungünstigen Eigenschaften stören stark bei der Verwendung in Kunststoffen bzw. Lacken, wie Automobillacken.

Um den heutigen Erfordernissen zu genügen, muß eine Verbindung eine ausgezeichnete Verträglichkeit mit bzw. eine hohe Löslichkeit in zahlreichen polymeren Substraten sowie eine hohe thermische Beständigkeit aufweisen. Außerdem soll die Verbindung bei der Hochtemperaturverarbeitung nicht aus den Kunststoffen austreten. Besonders die Entwicklung lösungsmittelarmer Beschichtungsformulierungen (z.B. High solids-Lacke) erfordert eine gute Löslichkeit der Stabilisatoren in den entsprechenden Lösungsmitteln. Weiterhin wird für eine Anwendung in Polyurethanen eine gute Löslichkeit insbesondere in den Polyetherol- bzw. Polyesterol-Komponenten gefordert.

Gegenstand der vorliegenden Erfindung sind Verbindungen der Formel I

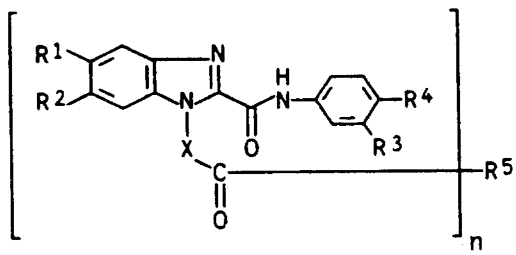

(I)

in der

$R^1$ und $R^2$ unabhängig voneinander für H, Cl, $C_1$- bis $C_4$-Alkyl, $C_1$- bis $C_4$-Alkoxy, für gegebenenfalls durch $C_1$- bis $C_4$-Alkyl oder $C_1$- bis $C_4$-Alkoxy substituiertes Phenyl oder $C_7$- bis $C_9$-Phenalkyl,

$R^3$ und $R^4$ unabhängig voneinander für H, $C_1$- bis $C_{18}$-Alkyl, durch -O- ein- oder mehrfach unterbrochenes $C_3$- bis $C_{18}$-Alkyl, $C_1$- bis $C_{18}$-Alkoxy, durch -O- ein- oder mehrfach unterbrochenes $C_4$- bis $C_{18}$-Alkoxy, gegebenenfalls durch $C_1$- bis $C_4$-Alkyl oder durch $C_1$- bis $C_4$-Alkoxy substituiertes Phenyl, $C_7$- bis $C_{15}$-Phenylalkyl, Phenoxy, $C_7$- bis $C_{15}$-Phenylalkyloxy, für $C_1$- bis $C_4$-Hydroxyalkyl, $C_1$- bis $C_4$-Hydroxyalkoxy, $C_1$- bis $C_{12}$-Alkylcarbonylamino, $C_1$- bis $C_{12}$-Alkanoyloxy, Benzoylamino oder Benzoyloxy oder $R^3$ und $R^4$ zusammen für Methylendioxy oder Ethylendioxy,

X für $C_1$- bis $C_5$-Alkylen,

n für 1 oder 2 und

$R^5$

a) - wenn n = 1 ist - für Cl, $-OR^6$ oder

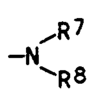

oder

b) - wenn n = 2 ist - für einen zweiwertigen Rest der Formeln -O-$R^{12}$-O- oder

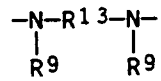

stehen,

worin

$R^6$ H, gegebenenfalls durch 1 bis 3 OH-Gruppen oder durch -O-$COR^9$ substituiertes $C_1$-$C_{18}$-Alkyl, durch -O-, -$NR^9$- ein- oder mehrfach unterbrochenes $C_3$-$C_{18}$-Alkyl, das gegebenenfalls durch -OH substituiert ist, gegebenenfalls durch OH substituiertes $C_5$-$C_{12}$-Cycloalkyl, gegebenenfalls durch OH substituiertes $C_2$-$C_{18}$-Alkenyl, $C_7$-$C_{15}$-Phenylalkyl, Phenoxyethyl,

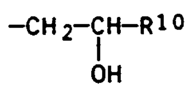 oder 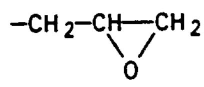

$R^7$ und $R^8$ unabhängig- voneinander H, $C_1$-$C_{18}$-Alkyl, durch O, -$NR^9$-ein- oder mehrfach unterbrochenes $C_3$-$C_{18}$-Alkyl, $C_5$-$C_{12}$-Cycloalkyl, Phenyl, Naphthyl, $C_3$-$C_{18}$-Alkenyl, $C_7$-$C_{15}$-Phenylalkyl, $C_2$-$C_4$-Hydroxyalkyl oder

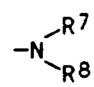

Pyrrolidinyl, Piperidinyl, Piperazinyl oder Morpholinyl,

$R^9$ H, $C_1$-$C_{18}$-Alkyl, $C_5$-$C_{12}$-Cycloalkyl, $C_3$-$C_8$-Alkenyl, Decenyl, Oleyl, Phenyl, Naphthyl oder $C_7$-$C_{18}$-Phenylalkyl,

$R^{10}$ H, $C_1$- bis $C_{18}$-Alkyl, $C_7$-$C_{18}$-Phenylalkyl, gegebenenfalls substituiertes Phenyl oder -$CH_2OR^{11}$,

$R^{11}$ $C_1$- bis $C_{18}$-Alkyl, $C_3$- bis $C_{18}$-Alkenyl, $C_5$- bis $C_{12}$-Cycloalkyl, Phenyl, $C_7$- bis $C_{15}$-Phenylalkyl,

$R^{12}$ $C_2$- bis $C_8$-Alkylen, $C_4$- bis $C_8$-Alkenylen, Cyclohexylen, durch -O-ein-oder mehrfach unterbrochenes $C_4$- bis $C_{18}$-Alkylen,

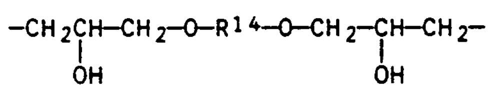 oder 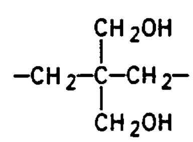 ,

$R^{13}$ gegebenenfalls durch -O- ein- oder mehrfach unterbrochenes $C_2$-bis $C_{12}$-Alkylen, Cyclohexylen,

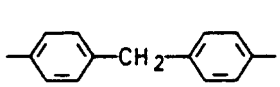 oder 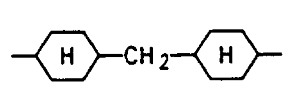 oder

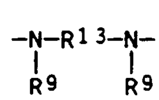

einen 2-wertigen Piperazinrest, und

$R^{14}$ $C_2$- bis $C_8$-Alkylen, durch -O- ein- oder mehrfach unterbrochenes $C_4$-bis $C_{18}$-Alkylen, 1,3- oder 1,4-Cyclohexylen, 1,3- oder 1,4-Phenylen,

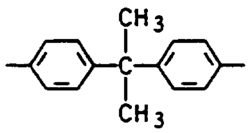 oder 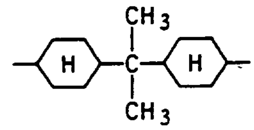 bedeuten.

Die erfindungsgemäßen Verbindungen der Formel (I) sind wirksame Lichtstabilisatoren für organische Materialien, so z.B. für eine große Anzahl von Polymeren. Sie werden nach üblichen Verfahren in die Polymeren eingearbeitet.

Die Benzimidazol-2-carbonsäureanilide (I) der Erfindung sind in Polymeren wie Kunststoffen und Lackbindemittel gut bis sehr gut löslich und sind dementsprechend hervorragend mit diesen Medien verträglich. Sie zeichnen sich außerdem durch geringe Flüchtigkeit und durch geringe Neigung zur Kristallisation aus. Die Verbindungen (I) sind weiterhin bei den zur Einarbeitung erforderlichen Temperaturen stabil.

Für $R^1$ und $R^2$ sind im einzelnen z.B. zu nennen: Wasserstoff, Chlor, Methyl, Ethyl, n-und i-Propyl, n-Butyl, i-Butyl, t-Butyl, Methoxy, Ethoxy, Propoxy und Butoxy, sowie Phenyl, Benzyl, $\alpha$-Methylbenzyl und $\alpha,\alpha$-Dimethylbenzyl.

Vorzugsweise stehen $R^1$ und $R^2$ für Wasserstoff, Methyl, Methoxy und Ethoxy.

Für $R^3$ und $R^4$ sind Wasserstoff, $C_1$- bis $C_{12}$-Alkyl, durch -O-einfach oder mehrfach unterbrochenes $C_3$- bis $C_{10}$-Alkyl, $C_1$- bis $C_{12}$-Alkoxy, durch -O-ein oder mehrfach unterbrochenes $C_4$- bis $C_{12}$-Alkoxy, $C_7$- bis $C_9$-Phenylalkyl, Phenoxy, $C_7$- bis $C_9$-Phenylalkoxy, $C_1$- bis $C_{12}$-Alkylcarbonylamino, Benzoylamino, $C_1$- bis $C_{12}$-Alkanoyloxy und Benzoyloxy bevorzugt.

Für $R^3$ und $R^4$ sind zusammen Methylendioxy oder Ethylendioxy zu nennen.

Neben den bestimmt genannten Substituenten für $R^3$ und $R^4$ sind im einzelnen z.B. zu nennen:

$\alpha$) $C_1$-$C_{12}$-Alkyl wie Methyl, Ethyl, n- und i-Propyl, n-Butyl, 2-Butyl, tert.-Butyl, n-Pentyl, tert.-Pentyl, n-Hexyl, n-Heptyl, n-Octyl, 2-Ethyl-hexyl, n-Nonyl, n-Decyl und n-Dodecyl;

$\beta$) $C_1$-$C_{12}$-Alkoxy wie Methoxy, Ethoxy, i-Propoxy, n-Butoxy, n-Pentoxy, i-Pentoxy, n-Hexoxy, n-Heptoxy, n-Octoxy, 2-Ethylhexoxy, n-Nonoxy, n-Decoxy und n-Dodecoxy;

$\gamma$) Phenylalkoxy wie Benzyloxy, 2-Phenylethoxy, 3-Phenylpropoxy.

Besonders bevorzugt sind Verbindungen I, bei denen

$R^3$ für H und

$R^4$ für $C_1$- bis $C_{12}$-Alkyl, $C_1$- bis $C_{12}$-Alkoxy, Phenoxy, $C_1$- bis $C_{12}$-Alkylcarbonylamino, Benzoylamino, $C_1$- bis $C_{12}$-Alkanoyloxy stehen.

X steht vorzugsweise für Methylen, Ethylen, n-Propylen, n-Butylen, n-Pentylen, i-Propylen, 1-Methylpropylen oder 1-Methylbutylen, insbesondere für Methylen, Ethylen oder Propylen.

Die Bedeutung von $R^5$ ist davon abhängig, ob n für 1 oder 2 steht.

Als $C_1$-$C_{18}$-Alkyl kommen für $R^6$, $R^7$, $R^8$, $R^9$, $R^{10}$ und $R^{11}$ z.B. in Betracht Methyl, Ethyl, i-Propyl, n-Butyl, sec.-Butyl, t-Butyl, t-Amyl, n-Hexyl, 2-Ethylhexyl, n-Octyl, 1,1,3,3-Tetramethylbutyl, n-Decyl, n-Dodecyl, 1,1,7,7-Tetramethyloctyl, n-Octadecyl.

Für $R^6$, $R^7$, $R^8$, $R^9$ und $R^{11}$ sind als $C_5$- bis $C_{12}$-Cycloalkyl z.B. zu nennen: Cyclopentyl, Cyclohexyl, Cycloheptyl, Cyclooctyl oder Cyclodecyl. Im Falle von $R^6$ kann der Cycloalkylrest auch durch OH substituiert sein.

$R^7$, $R^8$ und $R^9$ können z.B. folgende Alkenylreste bedeuten: Allyl, Methallyl, 2-n-Hexenyl, 4-n-Octenyl, Oleyl, Decenyl.

Für $R^6$ kommen als Alkenyl z.B. die für $R^7$, $R^8$ und $R^9$ genannten Alkenylreste, weiterhin Vinyl, 10-n-Undecenyl und 9-n-Octadecenyl in Betracht, wobei die für $R^6$ genannten Reste auch durch OH-substituiert sein können.

Als $C_3$- bis $C_{18}$-Alkyl, welches durch -O- oder -$NR^9$- unterbrochen und gegebenenfalls durch OH substituiert ist, sind für $R^6$, $R^7$ und $R^8$ z.B. im einzelnen zu nennen: Methyloxyethyl, Ethoxyethyl, Butoxyethyl, Butoxypropyl, $CH_3OCH_2CH_2OCH_2CH_2$-, $CH_3CH_2OCH_2CH_2OCH_2CH_2$-, $C_4H_9OCH_2CH_2OCH_2CH_2$-, Dodecyloxypropyl, 2-Hydroxyethyl, 2-Hydroxypropyl, 4-Hydroxybutyl, 6-Hydroxyhexyl, -$CH_2CH_2$-NH-$C_4H_9$, -$CH_2CH_2CH_2$-NH-$C_8H_{17}$,

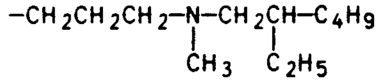 und 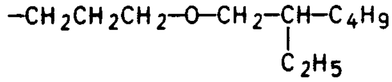 .

$R^6$ kann außerdem auch Phenoxyethyl sein.

Bedeuten

$R^7$ und $R^8$ $C_2$-$C_4$-Hydroxyalkyl, so können es die folgenden Reste sein: Hydroxyethyl, Hydroxypropyl, Hydroxybutyl.

$R^7$ und $R^8$ können z.B. folgende $C_7$- bis $C_{15}$-Phenylalkylreste bedeuten: Benzyl, 1-Phenylethyl, 2-Phenylethyl, 1-Phenylpropyl, $\alpha,\alpha$-Dimethylbenzyl.

Als Phenylalkyl kommen für $R^6$, $R^9$, $R^{10}$ und $R^{11}$ die für $R^7$ und $R^8$ genannten Reste in Betracht, wobei diese Reste gleich oder verschieden sein können.

$R^7$, $R^8$ und $R^9$ können außerdem für Phenyl, $\alpha$-Naphthyl oder $\beta$-Naphthyl stehen.

Als $C_2$- bis $C_8$-Alkylen kommen für $R^{12}$ und $R^{14}$ z.B. in Betracht: Ethylen, Propylen, Butylen, Hexylen und Octylen.

Für $R^{13}$ kommen als $C_2$-$C_{12}$-Alkylen z.B. die für $R^{12}$ und $R^{14}$ genannten Alkylenreste und Decylen und Dodecylen in Betracht.

Als $C_4$- bis $C_8$-Alkenylen kommt für $R^{12}$ z.B. Butenylen in Betracht.

Für $R^{12}$ und $R^{14}$ sind als durch -O- unterbrochenes $C_4$- bis $C_{18}$-Alkylen z.B. zu nennen:

$-CH_2CH_2OCH_2CH_2-$ ,

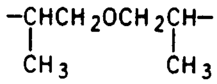 ,

$-CH_2CH_2OCH_2CH_2OCH_2CH_2-$ , $-CH_2CH_2OCH_2CH_2OCH_2CH_2OCH_2CH_2-$ ,

$-CH_2CH_2O[CH_2CH_2O]_3CH_2CH_2-$ ,

$-CH_2CH_2O[CH_2CH_2O]_4CH_2CH_2-$ , $-CH_2CH_2O[CH_2CH_2O]_5CH_2CH_2-$ ,

$-CH_2CH_2O[CH_2CH_2O]_6CH_2CH_2-$ und $-CH_2CH_2O[CH_2CH_2O]_7CH_2CH_2-$ .

Besonders bevorzugt sind Verbindungen der Formel (I), in der $R^1$, $R^2$, $R^3$, $R^4$ und X für die vorstehend als bevorzugt genannten Reste und

n für 1 und

$R^5$ für $-OR^6$ oder

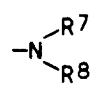

stehen, wobei

$R^6$ geradkettiges oder verzweigtes $C_1$- bis $C_{12}$-Alkyl, durch -O-unterbrochenes, geradkettiges oder verzweigtes $C_3$- bis $C_{18}$-Alkyl, Phenoxyethyl, Cyclohexyl oder

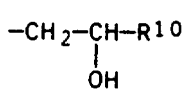 ,

$R^7$ und $R^8$ unabhängig voneinander geradkettiges oder verzweigtes $C_4$- bis $C_{12}$-Alkyl, durch -O-unterbrochenes, geradkettiges oder verzweigtes $C_3$- bis $C_{18}$-Alkyl oder Cyclohexyl,

$R^{10}$ geradkettiges oder verzweigtes $C_1$- bis $C_{12}$-Alkyl, oder $-CH_2OR^{11}$ und

$R^{11}$ geradkettiges oder verzweigtes $C_1$- bis $C_{12}$-Alkyl oder Cyclohexyl bedeuten.

Weiterhin sind solche Verbindungen der Formel (I) besonders bevorzugt, in der $R^1$, $R^2$, $R^3$, $R^4$ und X für die vorstehend als bevorzugt genannten Reste und

n für 2 und

$R^5$ für einen zweiwertigen Rest der Formeln -O-$R^{12}$-O- oder

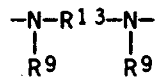

stehen, worin

$R^9$ H oder $C_1$- bis $C_8$-Alkyl,

$R^{12}$ $C_2$- bis $C_6$-Alkylen, durch -O- unterbrochenes $C_4$- bis $C_{10}$-Alkylen oder

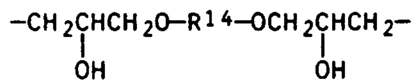 ,

$R^{13}$ geradkettiges oder verzweigtes $C_2$- bis $C_{12}$-Alkylen, und

$R^{14}$ $C_2$- bis $C_6$-Alkylen oder durch -O- unterbrochenes $C_4$- bis $C_{10}$-Alkylen bedeuten, wobei die gegebenenfalls durch -O- unterbrochenen Alkylenreste linear oder verzweigt sein können.

Bei der Herstellung der erfindungsgemäßen Verbindungen der Formel (I) geht man vorzugsweise von Benzimidazol-2-carbonsäureaniliden der Formel (II) aus, deren Darstellung z.B. in der EP-A-0 284 828 beschrieben ist.

Die Synthese der Benzimidazolcarbonsäuren der Formel (I) mit $R^6$ = H kann nach Weg a) durch Umsetzung mit Halogenalkylencarbonsäuren der Formel (III) oder nach Weg b) durch Reaktion mit Lactonen der Formel (IV), in der X z.B. für $C_2$-$C_5$-Alkylen wie Ethylen, Propylen, Butylen oder Pentylen steht, erfolgen.

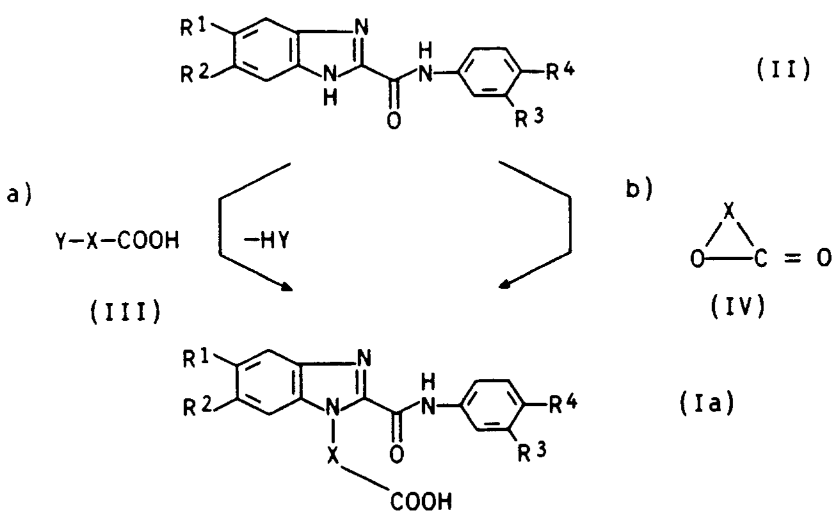


Der Rest Y steht vorzugsweise für Br, und insbesondere für Cl. Die Umsetzungen nach a) werden bevorzugt in inerten Lösungsmitteln wie Dimethylformamid, Dimethylacetamid, N-Methylpyrrolidon oder Ethylenglykoldiethern unter Verwendung einer Hilfsbase, wie $K_2CO_3$, $Na_2CO_3$, $KHCO_3$, $NaHCO_3$, bei Temperaturen zwischen 60 und 140 °C, vorzugsweise zwischen 80 und 120 °C, durchgeführt.

Die Umsetzung nach b) erfolgt vorzugsweise in einem Überschuß des Lactons (IV) in Gegenwart von basischen Verbindungen, wie NaOH, KOH, $Na_2CO_3$ oder $K_2CO_3$, bei Temperaturen zwischen 150 und 200 °C.

Die die nach obigen Verfahren erhältlichen Benzimidazolcarbonsäuren (Ia) können nach folgenden Verfahren in Verbindungen (I) mit $R^6$ ≠ H überführt werden:

1) Veresterung der Carbonsäure (Ia) mit Alkoholen der Formel $R^6OH$, wobei der Rest

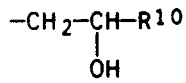

ausgenommen ist, in Gegenwart von Thionylchlorid, wobei man Verbindungen (I) mit n = 1 erhält.

Verwendet man Diole $HO-R^{12}-OH$, wobei für $R^{12}$ der Rest

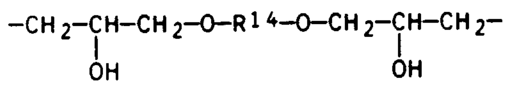

ausgenommen ist, so erhält man Verbindungen (I) mit n = 2.

2) Umsetzung der Benzimidazolcarbonsäuren (Ia) mit einem Epoxid (V) nach folgendem Formelschema:

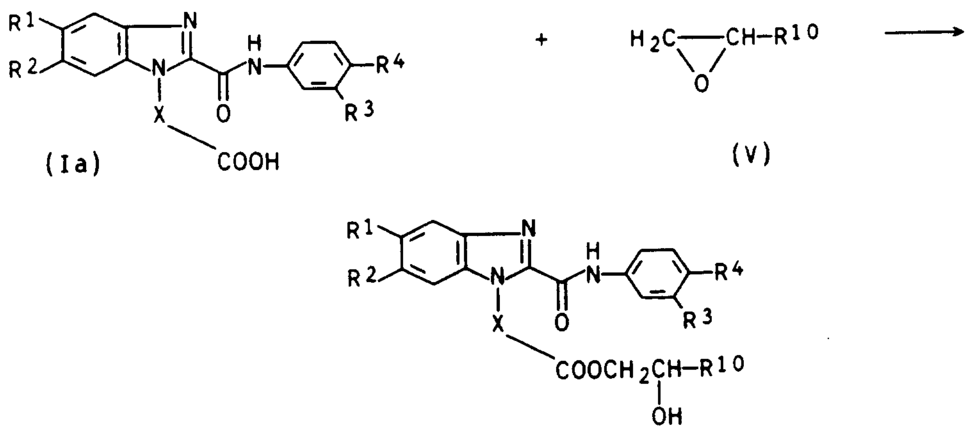

(Ia) (V)

3) Überführung der Benzimidazolcarbonsäuren (Ia) nach bekannten Verfahren in die Säurechloride der Formel I ($R^5$ = Cl, n = 1). Hierzu suspendiert man die entsprechende Benzimidazolcarbonsäure (Ia) z.B. in Toluol in Gegenwart einer katalytischen Menge Dimethylformamid, gibt den 2,5-fachen Überschuß an Thionylchlorid zu und rührt bei 80 °C bis zum Ende der Reaktion. Das Säurechlorid fällt aus und wird abgesaugt und mit Ligroin gewaschen.

Als Ausgangsverbindungen für die Herstellung weiterer Verbindungen (I) kommen auch die nach 1) erhältlichen Benzimidazol-1-alkylester, insbesondere die mit niedermolekularen Alkylgruppen, wie Methyl und Ethyl, in Frage, die auch nach folgenden Verfahren hergestellt werden können:

1a) Umsetzung von Benzimidazol-2-carbonsäureaniliden (II) mit Halogenalkylencarbonsäureester der Formel (VI)

$Y-X-COOR^6$ (VI),

oder im Falle von X = $-CH_2CH_2-$ durch Umsetzen mit Acrylsäurealkylestern der Formel (VII)

$H_2C = CH-COOR^6$ (VII).

Die so erhältlichen niedermolekularen Benzimidazol-1-carbonsäureester (I) können nach folgenden Verfahren weiter umgesetzt werden.

4) Umesterung nach 1) oder 1a) erhältlichen Benzimidazol-1-carbonsäuremethylester (I) im Falle von n = 1 mit Alkoholen der Formel $R^6$-OH in Gegenwart eines Umesterungskatalysators, wie Tetrabutylorthotitanat, Li-Amid, Na-Methylat oder p-Toluolsulfonsäure, unter Abdestillieren des gebildeten Methanols. Man erhält so höhermolekulare Benzimidazol-1-carbonsäureester (I).

In analoger Weise können auch Umesterungen der nach 1) oder 1a) erhältichen Ester (I) mit Diolen der Formel HO-$R^{12}$-OH durchgeführt werden, wobei Verbindungen (I) mit n = 2 erhalten werden. Die Molmenge an Diol wird entsprechend der Reaktionsgleichung auf die Hälfte herabgesetzt.

Zur Herstellung der Benzimidazol-1-carbonsäureamide der Formel I geht man folgendermaßen vor:

5) Umsetzung von Benzimidazol-1-carbonsäureestern, bevorzugt Methyl- oder Ethylester, gegebenenfalls in Gegenwart von Katalysatoren, z.B. Li-Amid oder Na-Methylat, mit Ammoniak oder einem primären oder sekundären Amin der Formel

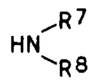

oder mit Diaminen der Formel

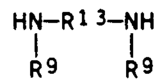

jeweils unter Abdestillieren des freiwerdenden Alkohols. Im ersteren Fall erhält man (I) mit n = 1 und im letzteren Verbindungen (I) mit n = 2.

Eine weitere Herstellungsmöglichkeit der Benzimidazol-1-carbonsäureamide besteht darin Benzimidazol-2-carbonsäuranilide (II) mit einem Halogencarbonsäureamid der Formel (VIII)

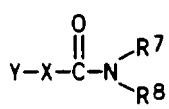

(VIII)

oder mit einem Dicarbonsäureamid der Formel (IX)

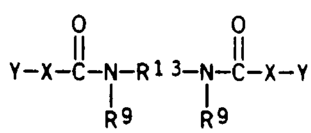

(IX)

umzusetzen. Mit (VIII) erhält man Verbindungen (I) mit n = 1 und mit (IX) Verbindungen (I) mit n = 2.

Die Amide der Formel (VIII) und (IX) können nach bekannten Verfahren, beispielsweise durch Reaktionen der entsprechenden Halogencarbonsäurechloride mit den oben beschriebenen primären oder sekundären Mono- oder Diaminen, hergestellt werden.

6) Die Benzimidazol-1-carbonsäuren (Ia) können über im Lack enthaltende Bindemittel in PES-Harze oder Epoxidharze eingebaut und verankert werden. Der Einbau ist sowohl bei der Herstellung der Harze als auch bei der chemischen Vernetzung der Lackharze beim Einbrennvorgang möglich.

Die Einarbeitung kann beispielsweise durch Einmischen der Verbindungen (I) und gegebenenfalls weiterer Additive in die Schmelze nach in der Technik üblichen Verfahren vor oder während der Formgebung, oder auch durch Aufbringen der gelösten oder dispergierten Verbindungen auf das Polymere direkt oder durch Einmischen in eine Lösung, Suspension oder Emulsion des Polymeren, wobei gegebenenfalls das Lösungsmittel nachträglich entfernt werden kann, z.B. durch Verdunsten erfolgen.

Als Polymere kommen z.B. in Betracht: Polyolefine, Polystyrol, Styrolpolymerisate, halogenhaltige Vinylpolymere, Polyacrylate, Polymethacrylate, Polyacrylamide, Polyacrylnitril, Polyvinylalkohol und dessen Acylderivate, Polyacetate, Polyalkylenoxide, Polyphenylenoxide, Polyurethane und Polyharnstoffe, Polysulfone, Polyamide, Polyester, Polycarbonate, vernetzte Polymere aus Aldehyden und Phenolen, Harnstoffe oder Melamin, ungesättigte Polyesterharze, Alkyldharze, duroplastische und thermoplastische Acrylharze.

Gegenstand dieser Erfindung sind somit auch stabilisierte organische Materialien, insbesondere synthetische Polymere, welche 0,05 bis 10, vorzugsweise 0,1 bis 5 Gew.-% einer oder mehrerer Verbindungen der Formel (I) enthalten (bezogen auf das organische Material).

Die erfindungsgemäßen Verbindungen sind nicht nur wirksame Lichtschutzmittel, sondern aufgrund ihrer geringen Flüchtigkeit bei hoher Temperatur auch hervorragend zum Stabilisieren von Polymeren geeignet, welche bei hohen Temperaturen verarbeitet werden müssen.

Die erfindungsgemäßen Verbindungen (I) sind dementsprechend hervorragend zum Stabilisieren von Polyestern wie Polyethylenterephthalat, Polybutylenterephthalat oder deren Copolymeren, von Polyamiden, z.B. Nylon-6, Nylon-6,6, Nylon-6,10 u.a. sowie von Copolymeren von MF- und UF-Harzen, von heißvernetzbaren und thermoplastischen Acrylharzen und von Polyurethanen geeignet.

Ganz besonders geeignet sind Verbindungen (I) zum Stabilisieren von Lacken, die derartige Polymere enthalten.

Die mit (I) stabilisierten Materialien können nach bekannten Verfahren in die für die Anwendung üblichen Formen überführt werden, z.B. zu Folien, Fasern, Bändchen, Profile, oder als Bindemittel für Lacke, Klebstoffe, Kitte oder Formmassen verwendet werden.

In der Praxis können die Verbindungen der Formel I zusammen mit 0,1 bis 5, vorzugsweise 0,5 bis 3 Gew.-% an weiteren üblichen Zusatzstoffen, wie Antioxidantien, weitere Lichtstabilisatoren, oder Gemischen davon angewendet werden.

Solche üblichen Zusatzstoffe sind z.B.: Antioxidantien, UV-Absorber und Lichtschutzmittel wie 2-(2'-Hydroxyphenyl)-benztriazole, 2,4-Bis-(2'-hydroxyphenyl)-6-alkyl-s-triazine, 2-Hydroxybenzophenone, 1,3-Bis-(2'-hydroxybenzoyl)-benzole, Ester von gegebenenfalls substituierten Benzoesäuren, Acrylate, außerdem Nickelverbindungen, sterisch gehinderte Amine, Metalldesaktivatoren, Phosphite, peroxidzerstörende Verbindungen, Polyamidstabilisatoren, basische Co-Stabilisatoren, Nukleierungsmittel und sonstige Zusätze wie Weichmacher, Gleitmittel, Emulgatoren, Füllstoffe, Ruß, Kaolin, Talk, Glasfasern, Pigmente, optische Aufheller, Flammschutzmittel und Antistatica.

Die Verbindungen der Formel (I) eignen sich auch hervorragend zum Schutz von ABS-Polymeren und insbesondere von Polyurethanen, die sich von Polyethern, Polyestern und Polybutadien mit endständigen Hydroxylgruppen und aliphatischen oder aromatischen Polyisocyanaten ableiten, sowie deren Vorprodukte gegen Abbau durch Wärme- und insbesondere durch Lichteinwirkung.

Eine verbesserte stabilisierende Wirkung erzielt man, wenn man zusätzlich ein bekanntes Antioxidans, z.B. eine Verbindung auf der Basis sterisch gehinderter Phenole oder einen Schwefel oder Phosphor enthaltenden Costabilisator verwendet.

Als derartiger phenolische Antioxidationsmittel kommen z.B. 2,6-Di-tert.-butyl-4-methylphenol, n-Octadecyl-$\beta$-(3,5-di-tert.-butyl-4-hydroxyphenyl)-propionat, 1,1,3-Tris-(2-methyl-4-hydroxy-5-tert.-butyl-phenyl)-butan, 1,3,5-Trimethyl-2,4,6-tris-(3,5-di-tert.-butyl-4-hydroxy-benzyl)-benzol, 1,3,5-Tris-(3,5-di-tert.-butyl-4-hydroxybenzyl)-isocyanurat, 1,3,5-Tris-[3,5-di-tert.-butyl-4-hydroxyphenyl-propionyloxy-ethyl]-isocyanurat, 1,3,5-Tris-(2,6-di-methyl-3-hydroxy-4-tert.-butylbenzyl)-isocyanurat, Pentaerythrit-tetrakis-[-(3,5-di-tert.-butyl-4-hydroxy-phenyl)-propionat] in Betracht.

Als phosphorhaltige Antioxidantien sind z.B. zu nennen: Tis-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit.

Als Schwefel enthaltende Antioxidationsmittel sind z.B. Dilaurylthiodipropionat, Dimyristylthiodipropionat, Distearylthiodipropionat, Pentaerythrittetrakis-($\beta$-laurylthiopropionat), Pentaerythrittetrakis-($\beta$-hexyl-thiopropionat) zu nennen.

Eine besonders gute Stabilisierung erhält man, wenn man zu den Verbindungen der Formeln (I) noch mindestens einen Lichtstabilisator aus der Verbindungsklasse der sterisch gehinderten Amine in üblicher Konzentration zusetzt.

Als sterisch gehinderte Amine kommen z. B. in Betracht: Bis-(2,2,6,6-tetramethylpiperidyl)-sebacat, Bis-(1,2,2,6,6-pentamethylpiperidyl)-sebacat, Bis(-(1,2,2,6,6-pentamethylpiperidyl)-ester, das Kondensationsprodukt von 1-Hydroxyethyl-2,2,6,6-tetramethyl-4-hydroxypiperidin und Bernsteinsäure, das Kondensationsprodukt von N,N'-(2,2,6,6-Tetramethylpiperidyl)-hexamethylendiamin und 4-tert.-Octylamino-2,6-dichlor-1,3,5-s-triazin, Tris-(2,2,6,6-Tetramethylpiperidyl)-nitrilotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Eine ganz besonders gute Stabilisierung von Polyurethanen erhält man, wenn das Polyurethan mit einem Gemisch aus mindestens einer Verbindung der Formel (I), mindestens einem der oben genannten Antioxidantien und mindestens einer der sterisch gehinderten Aminverbindungen stabilisiert wird.

Die Schwerflüchigkeit, insbesondere wenn diese mit guter Substratverträglichkeit und Löslichkeit kombiniert ist, erlaubt die unproblematische Einarbeitung der erfindungsgemäßen Verbindungen in das Polymer, das auch nach der Verarbeitung bei höheren Temperaturen hervorragend stabilisiert ist.

Die hohe Substratverträglichkeit der erfindungsgemäßen Verbindungen (I) zusammen mit der bereits diskutierten Schwerflüchtigkeit gestattet die Verarbeitung und Verwendung der stabilisierten Produkte bei höheren Temperaturen, sowie eine verlängerte Gebrauchsdauer der Produkte bei üblichen Temperaturen. Durch diese Eigenschaften wird das unerwünschte Ausschwitzen des Stabilisators während der Verarbeitung verhindert, was sonst häufig zu Schädigungen der Produktionsanlage führt.

Die folgenden Beispiele sollen die Erfindung zusätzlich erläutern.

A) Herstellungsbeispiele

Beispiel 1

112,4 g (0,4 Mol) 4'-Ethoxy-benzimidazol-2-carbonsäureanilid und 165,6 g $K_2CO_3$ werden in 600 ml Dimethylformamid suspendiert und auf 95 bis 100 °C erhitzt. Bei dieser Temperatur werden im Verlauf von 150 Minuten 57 g Chloressigsäure portionsweise in die Reaktionsmischung eingetragen und weitere 5 Stunden gerührt. Zur Aufarbeitung wird das Reaktionsgemisch in 3 l Wasser gegossen. Der pH-Wert wird durch Zugabe von $K_2CO_3$ auf 11 eingestellt, die ungelösten Bestandteile werden abfiltriert und das Filtrat durch Zugabe von konzentrierter Salzsäure bis zu einem pH-Wert von 1 angesäuert. Das ausgefallene Produkt wird abgesaugt und mit Wasser gut gewaschen. Es werden 78,4 g eines schwach gelben Feststoffes der Formel

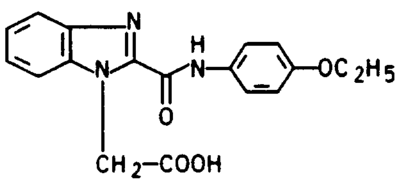

mit einem Schmelzpunkt von 234 - 236 °C (Zers.) erhalten.

| Analyse: $C_{18}H_{17}N_3O_4$ (339,4) | | | | |
|---|---|---|---|---|
| Ber.: | C 63,72 | H 5,02 | N 12,39 | O 18,87 |
| Gef.: | C 63,4 | H 5,1 | N 12,2 | O 18,7 |

Beispiel 2

Verwendet man in dem in Beispiel 1 beschriebenen Verfahren Chlorpropionsäure anstelle Chloressigsäure, so erhält man die entsprechende Benzimidazol-N-propionsäure der Formel

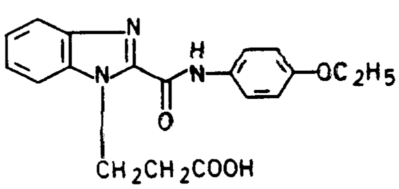

mit dem Schmelzpunkt 205 °C (Zers.)

Beispiel 3

140,5 g (0,5 Mol) 4'-Ethoxy-benzimidazol-2-carbonsäureanilid und 82,8 g $K_2CO_3$ werden in 600 ml $\gamma$-Butyrolacton suspendiert und 4 Stunden bei 150 °C gerührt. Anschließend wird die Reaktionsmischung auf

4 l Wasser gegossen, mit Aktivkohle versetzt und 1 Stunde nachgerührt. Nach dem Filtrieren wird unter Zusatz von Eis und durch Zugabe von konzentrierter Salzsäure (pH = 2 bis 3) die Carbonsäure ausgefällt, abgesaugt und mit Wasser gewaschen. Es werden 162 g eines farblosen Produktes der Formel

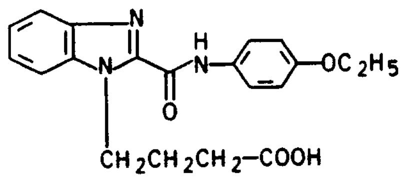

vom Schmp.: 172 bis 175 °C isoliert.

| Analyse: $C_{20}$ $H_{21}$ $N_3$ $O_4$ (367,4) | | | | |
|---|---|---|---|---|
| Ber.:<br>Gef.: | C 65,39<br>C 64,7 | H 5,73<br>H 5,9 | N 11,44<br>N 10,9 | O 17,44<br>O 18,3 |

Beispiel 4

338 g 4'-Ethoxy-benzimidazol-2-carbonsäureanilid, 200 g $K_2CO_3$ und 174 g Chloressigsäuremethylester werden in 800 ml Dimethylformamid 75 Minuten auf 90 bis 95 °C unter Rühren erhitzt. Die Reaktionsmischung wird dann heiß abgesaugt und mit ca. 200 ml heißem Dimethylformamid nachgewaschen. Die erhaltene Lösung wird mit Aktivkohle behandelt und in 10 l Methanol eingerührt. Der ausgefallene Niederschlag wird abgesaugt, mit Methanol gewaschen und bei 80 °C getrocknet. Man erhält 258 g des Methylesters mit der Formel

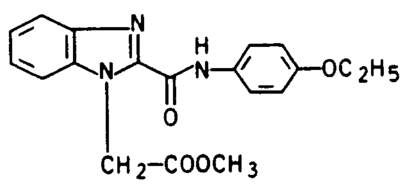

mit dem Schmp.: 140 - 141 °C.

| Analyse: $C_{19}$ $H_{19}$ $N_3$ $O_4$ (353,4) | | | | |
|---|---|---|---|---|
| Ber.:<br>Gef.: | C 64,58<br>C 64,6 | H 5,39<br>H 5,6 | N 11,90<br>N 11,9 | O 18,13<br>O 18,0 |

Beispiel 5

169 g 4'-Ethoxy-benzimidazol-2-carbonsäureanilid, 100 g $K_3CO_3$ und 98 g Chlorpropionsäuremethylester werden in 500 ml Dimethylformamid 3,5 Stunden auf 90 bis 95 °C erhitzt. Die Aufarbeitung erfolgt nach der in Beispiel 4 beschriebenen Verfahrensweise. Die Fällung des Produktes wird in 4 l Methanol unter Zusatz von 1 kg Eis durchgeführt. Nach Absaugen, Waschen und Trocknen werden 188,1 g eines farblosen Feststoffes der Formel

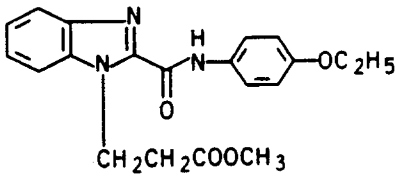

mit Schmp.: 121 - 122 ° C isoliert.

| Analyse: $C_{20}$ $H_{21}$ $N_3$ $O_4$ (367,4) | | | | |
|---|---|---|---|---|
| Ber.:<br>Gef.: | C 65,39<br>C 65,5 | H 5,73<br>H 5,8 | N 11,44<br>N 11,3 | O 17,44<br>O 17,5 |

Beispiel 6

Verwendet man Chlorbuttersäuremethylester anstelle von Chlorpropionsäuremethylester, so erhält man nach der in Beipiel 5 beschriebenen Verfahrensweise den Benzimidazol-N-buttersäure-methylester der Formel

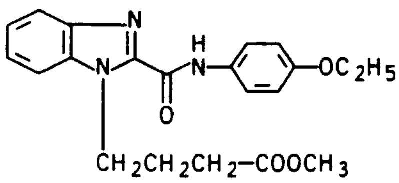

mit dem Schmp.: 110 - 111 ° C.

| Analyse: $C_{21}$ $H_{23}$ $N_3$ $O_4$ (381,4) | | | | |
|---|---|---|---|---|
| Ber.:<br>Gef.: | C 66,14<br>C 65,8 | H 6.04<br>H 6,1 | N 11,02<br>N 11,1 | O 16.80<br>O 16,8 |

Beispiel 7 - 14

Analog den in Beispiel 4 bis 5 beschriebenen Verfahrensweisen wurden die in Tabelle 1 aufgeführten Benzimidazol-1-carbonsäureester hergestellt.

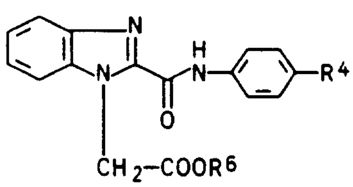

Tabelle 1

| Bsp. | $R^4$ | $R^6$ | Fp[°C] | Analyse | | | | |
|---|---|---|---|---|---|---|---|---|
| | | | | | C | H | N | O |
| 7 | H | $C_2H_5$ | 136 - 8 | ber.<br>gef. | 66,87<br>67,2 | 5,27<br>5,5 | 13,0<br>12,9 | 14,86<br>14,5 |
| 8 | $OCH_3$ | $CH_3$ | 170 - 1 | ber.<br>gef. | 63,72<br>63,5 | 5,02<br>5,2 | 12,38<br>12,5 | 18,88<br>18,5 |
| 9 | $OC_2H_5$ | $C_2H_5$ | 132 - 3 | ber.<br>gef. | 65,39<br>65,3 | 5,73<br>5,9 | 11,44<br>11,5 | 17,44<br>17,4 |
| 10 | $OC_2H_5$ | t-$C_4H_9$ | 145 - 8 | ber.<br>gef. | 66,83<br>67,1 | 6,34<br>6,4 | 10,63<br>10,4 | 16,20<br>16,0 |
| 11 | $OC_2H_5$ | n-$C_{12}H_{25}$ | 88 - 90 | ber.<br>gef. | 70,96<br>70,9 | 8,14<br>8,3 | 8,28<br>8,1 | 12,62<br>12,4 |
| 12 | $OC_2H_5$ | n-$C_{16}H_{33}$ | 82 - 83 | ber.<br>gef. | 72,41<br>72,7 | 8,76<br>8,8 | 7,46<br>7,3 | 11,36<br>11,4 |
| 13 | $OC_2H_5$ | n-$C_{18}H_{37}$ | 82 | ber.<br>gef. | 73,09<br>72,9 | 9,04<br>9,3 | 7,11<br>6,6 | 10,82<br>11,2 |
| 14 | $NHCOCH(C_2H_5)C_4H_9$ | t-$C_4H_9$ | 62 - 5 | ber.<br>gef. | 68,25<br>68,1 | 7,37<br>7,4 | 11,38<br>11,6 | 13,00<br>13,4 |

Beispiel 15

36,7 g (1-(3-Carboxypropyl)-4'-ethoxy-benzimidazol-2-carbonsäureanilid(Verbindung aus Bsp. 3) werden in 400 ml absoluten Ethanol suspendiert. Bei -5 bis 0°C tropft man 13,1 g Thionylchlorid zu, rührt 2 Stunden bei Raumtemperatur und weitere 4 Stunden bei 40 bis 45°C. Die Reaktionsmischung wird auf 1 l Eiswasser gegossen und mit $NaHCO_3$ neutralisiert. Der ausgefallene Feststoff wird abgesaugt und mit Wasser gut gewaschen. Man erhält 37,6 g des Ethylesters mit der Formel

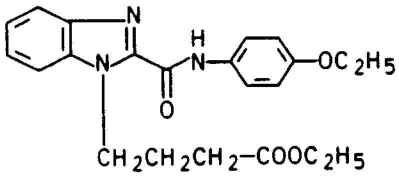

mit dem Schmp.: 94 - 95°C.

Beispiel 16

Zu einer Suspension von 36,7 g 1-(3-Carboxypropyl)-4'-ethoxy-benzimidazol-2-carbonsäureanilid in 300 ml 2-Ethylhexanol werden 13,1 g Thionylchlorid unter Eiskühlung zugetropft. Das Reaktionsgemisch wird 2 Stunden bei Raumtemperatur und 4 Stunden bei 50 bis 55°C gerührt. Die Suspension wird abgesaugt, mit Ligroin gewaschen, und der erhaltene Rückstand in 500 ml Essigsäureethylester gelöst. Die Lösung wird mit einer gesättigten $NaHCO_3$-Lösung und zweimal mit ca. 500 ml Wasser gewaschen, über $Na_2SO_4$ getrocknet und anschließend mit Aktivkohle und Bleicherde gereinigt. Nach dem Abdestillieren der Lösungsmittel verbleiben 41,0 g eines farblosen Produktes der Formel

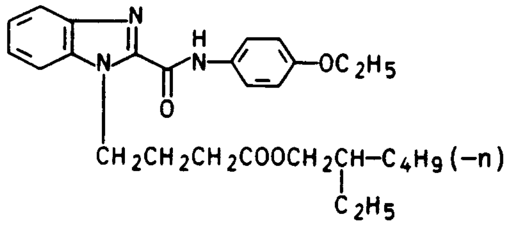

mit dem Schmp.: 56 - 60 ° C.

| Analyse: $C_{28}$ $H_{37}$ $N_3$ $O_4$ (395,5) | | | | |
|---|---|---|---|---|
| Ber.:<br>Gef.: | C 70,14<br>C 70,3 | H 7,73<br>H 7,9 | N 8,77<br>N 8,5 | O 13,36<br>O 13,4 |

Beispiel 17

17,7 g 1-Carbomethoxymethyl-4'-ethoxy-benzimidazol-2-carbonsäureanilid (Verbindung aus Beispiel 4) werden in 100 ml Isobutanol unter Zusatz von 0,4 g Natriummethylat 2 Stunden auf 100 bis 105 ° C erhitzt. Das überschüssige Isobutanol wird im Vakuum abgezogen, der Rückstand in Wasser aufgerührt und abgesaugt. Der erhaltene Feststoff wird in 400 ml Aceton gelöst, die Lösung mit Aktivkohle gereinigt und auf 1,5 l Eiswasser gegossen. Nach dem Absaugen, Waschen und Trocknen werden 13,5 g farbloses Produkt der Formel

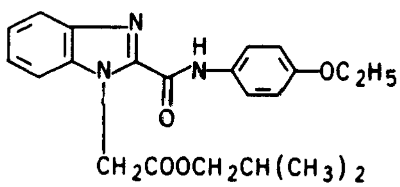

mit einem Schmelzpunkt von 80 - 84 ° C isoliert.

| Analyse: $C_{22}$ $H_{25}$ $N_3$ $O_4$ (395,5) | | | | |
|---|---|---|---|---|
| Ber.:<br>Gef.: | C 66,83<br>C 66,8 | H 6,33<br>H 6,5 | N 10,63<br>N 10,7 | O 16,21<br>O 16.0 |

Beispiel 18

17,7 g 1-Carbomethoxymethyl-4'-ethoxy-benzimidazol-2-carbonsäureanilid (Verbindung aus Bsp. 4) werden zusammen mit 80 ml 1-Octanol und 6 g p-Toluolsulfonsäure 7,5 Stunden auf 120 bsi 125 ° C erhitzt. Die Reaktionsmischung wird in 300 ml Toluol aufgenommen, dreimal mit 800 ml Wasser ausgeschüttelt, und die Toluolphase über $Na_2SO_4$ getrocknet. Nach dem Reinigen mit Aktivkohle und Bleicherde wird das Lösungsmittel und der überschüssige Alkohol im Vakuum abdestilliert. Man erhält 21,2 g eines schwach gelben Öles mit der Formel

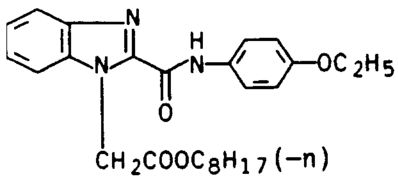

das bei längerem Stehen wachsartig erstarrt (Schmp.: 80 - 81 °C)

| Analyse: $C_{26}$ $H_{33}$ $N_3$ $O_4$ (451,6) | | | | |
|---|---|---|---|---|
| Ber.:<br>Gef.: | C 69,18<br>C 69,1 | H 7,32<br>H 7,2 | N 9,31<br>N 8,9 | O 14,19<br>O 14,0 |

Beispiel 19 - 23

Analog Beispiel 18 wurden die folgenden erfindungsgemäßen Ester durch Umesterung der entsprechenden Benzimidazol-1-carbonsäuremethylester mit den entsprechenden Alkoholen hergestellt:

Beispiel 19

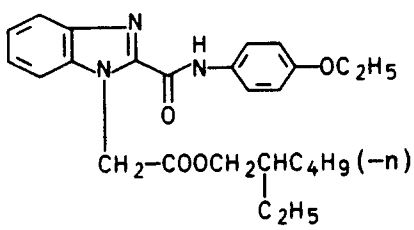

Farbloses Wachs Schmp.: 48 - 51 °C.

| Analyse: $C_{26}$ $H_{33}$ $N_3$ $O_4$ (451,6) | | | | |
|---|---|---|---|---|
| Ber.:<br>Gef.: | C 69,18<br>C 69,2 | H 7,32<br>H 7,5 | N 9,31<br>N 8,7 | O 14,19<br>O 14,0 |

Beispiel 20

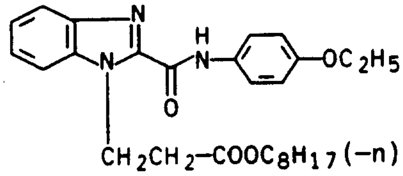

Schmelzpunkt: 77 - 79 °C.

| Analyse: $C_{27}$ $H_{35}$ $N_3$ $O_4$ (465,7) | | | | |
|---|---|---|---|---|
| Ber.: | C 69,67 | H 7,53 | N 9,03 | O 13,77 |
| Gef.: | C 69,4 | H 7,7 | N 8,8 | O 13,6 |

Beispiel 21

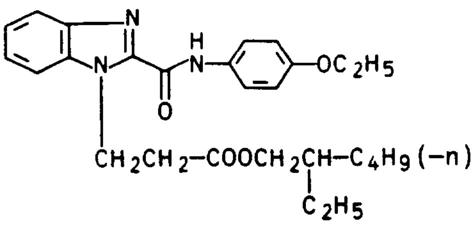

Schmp.: 67 - 69 °C.

| Analyse: $C_{27}$ $H_{35}$ $N_3$ $O_4$ (465,7) | | | | |
|---|---|---|---|---|
| Ber.: | C 69,67 | H 7,53 | N 9,03 | O 13,77 |
| Gef.: | C 69,5 | H 7,7 | N 8,9 | O 14,0 |

Beispiel 22

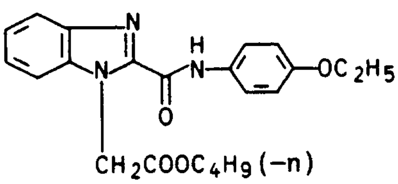

Schmelzpunkt: 108 - 109 °C.

| Analyse: $C_{22}$ $H_{25}$ $N_3$ $O_4$ (395,5) | | | | |
|---|---|---|---|---|
| Ber.: | C 66,82 | H 6,37 | N 10,63 | O 16,18 |
| Gef.: | C 66,9 | H 6,4 | N 10,6 | O 16,0 |

Beispiel 23

Eingesetzter Alkohol = 1 : 1-Gemisch aus Isobutanol und n-Butanol

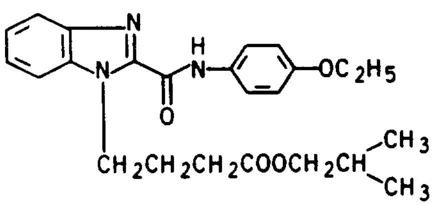

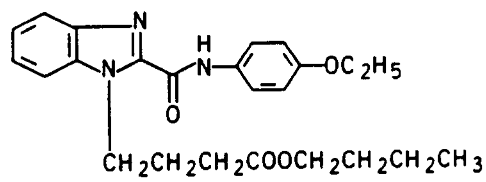

1 : 1 Gemisch ($^{1}H$-NMR-analytisch)

| Analyse: $C_{24}H_{29}N_3O_4$ (423,6) | | | | |
|---|---|---|---|---|
| Ber.: | C 68,08 | H 6,86 | N 9,93 | O 15,13 |
| Gef.: | C 68,0 | H 7,0 | N 9,9 | O 15,1 |

Beispiel 24

17,7 g 1-Carbomethoxymethyl-4'-ethoxy-benzimidazol-2-carbonsäureanilid und 6 g p-Toluolsulfonsäure werden in 60 ml Benzylalkohol 4 Stunden auf 135 bis 140 °C erhitzt. Die Reaktionsmischung wird auf 50 °C abgekühlt und in ca. 500 ml Methanol gegeben. Durch langsamen Zusatz von 250 ml Eiswasser wird der Benzylester ausgefällt und 15 Minuten nachgerührt. Das farblose Produkt wird abgesaugt, mit Methanol und Ligroin gewaschen. Man erhält 16,3 g eines Feststoffes der Formel

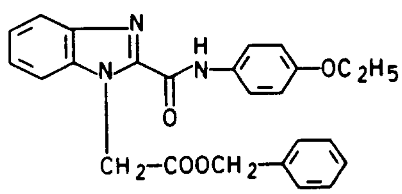

mit dem Schmp.: 130 - 131 °C.

| Analyse: $C_{25}H_{23}N_3O_4$ (429,6) | | | | |
|---|---|---|---|---|
| Ber.: | C 69,93 | H 5,37 | N 9,79 | O 14,91 |
| Gef.: | C 70,1 | H 5,6 | N 9,7 | O 14,5 |

Beispiel 25 - 29

Nach der in Beispiel 24 beschriebenen Verfahrensweise wurden durch Verwendung von 2-Phenylethanol, 2-Phenoxyethanol, Ethylenglykolethylether Diethylenglykolmethylether und Diethylenglykolbutylether die folgenden erfindungsgemäßen Benzimidazol-1-carbonsäureester hergestellt:

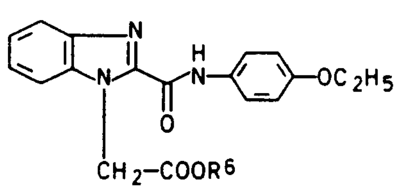

| Bsp. | $R^6$ | Fp[°C] | | Analyse C | H | N | O |
|---|---|---|---|---|---|---|---|
| 25 | $CH_2CH_2$ 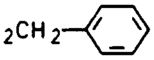 | 128 | ber.<br>gef. | 70,43<br>70,7 | 5,65<br>5,7 | 9,48<br>9,6 | 14,44<br>14,3 |
| 26 | $CH_2CH_2$ 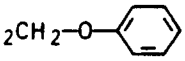 | 99 - 100 | ber.<br>gef. | 67,97<br>67,9 | 5,45<br>5,7 | 9,15<br>9,2 | 17,43<br>17,9 |
| 27 | $CH_2CH_2OC_2H_5$ | 88 - 90 | ber.<br>gef. | 64,23<br>64,6 | 6,09<br>6,2 | 10,22<br>10,0 | 19,46<br>19,5 |
| 28 | $CH_2CH_2OCH_2CH_2OCH_3$ | 98 - 99 | ber.<br>gef. | 62,58<br>62,7 | 6,13<br>6,3 | 9,52<br>9,5 | 21,77<br>21,5 |
| 29 | $CH_2CH_2OCH_2CH_2OC_4H_9$(-n) | 53 | ber.<br>gef. | 64,6<br>64,3 | 6,84<br>7,1 | 8,69<br>8,6 | 19,87<br>19,9 |

Beispiel 30

17,0 g 1-Carboxymethyl-4'-ethoxy-benzimidazol-2-carbonsäureanilid (aus Beisp. 1) und 2,7 g Diethylenglykol werden unter Zusatz von 5 g p-Toluolsulfonsäure in 200 ml Toluol 16 Stunden unter Rückfluß erhitzt. Das Lösungsmittel wird im Vakuum abdestilliert, der Rückstand in 500 ml Methanol heiß gelöst und mit Aktivkohle gereinigt. Unter Zusatz von 400 ml Eiswasser wird das Produkt gefällt, abgesaugt und mit Wasser gewaschen. Es werden 10,5 g eines schwach gelben Produktes der Formel

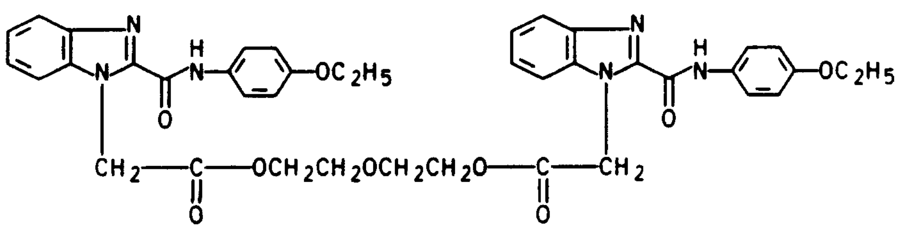

mit dem Schmp.: 160 °C isoliert.

| Analyse: $C_{40}H_{40}N_6O_9$ (748,9) | | | | |
|---|---|---|---|---|
| Ber.:<br>Gef.: | C 64,17<br>C 63,9 | H 5,35<br>H 5,4 | N 11,23<br>N 11,3 | O 19,23<br>O 19,1 |

Beispiel 31

38,1 g 1-Carbomethoxypropyl-4'-ethoxy-benzimidazol-2-carbonsäureanilid (Verbindung aus Beisp. 6) und 12 g p-Toluolsulfonsäure werden in 100 ml Triethylenglykol 4,5 Stunden auf 140 bis 145 °C erhitzt. Anschließend gießt man die Reaktionsmischung auf 1 l Eiswasser und extrahiert die wässrige Phase mit zweimal 500 ml Essigester. Die organische Phase wird mit Wasser gewaschen, über $Na_2SO_4$ getrocknet und mit Aktivkohle gereinigt. Nach dem Abdestillieren des Lösungsmittels im Vakuum erhält man 41 g eines Produktes der Formel

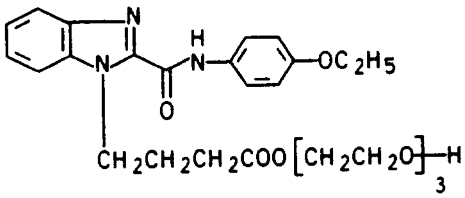

in Form eines hellgelben Öles.

| Analyse: $C_{26}$ $H_{33}$ $N_3$ $O_7$ (499,6) | | | | |
|---|---|---|---|---|
| Ber.: | C 62,52 | H 6,62 | N 8,42 | O 22,44 |
| Gef.: | C 62,7 | H 6,7 | N 8,4 | O 22,5 |

Beispiel 32 - 36

Analog Beispiel 31 wurden durch Umestern der entsprechenden Benzimidazol-carbonsäuremethylester mit Polyglykolen und Glykolethern folgende höhermolekulare Benzimidazol-1-carbonsäureester hergestellt:

Beispiel 32

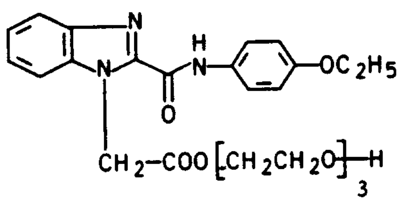

Hellgelbes Harz.

| Analyse: $C_{24}$ $H_{29}$ $N_3$ $O_7$ (471,5) | | | | |
|---|---|---|---|---|
| Ber.: | C 61,14 | H 6,17 | N 8,91 | O 23,78 |
| Gef.: | C 61,1 | H 6,2 | N 9,0 | O 23,9 |

Beispiel 33

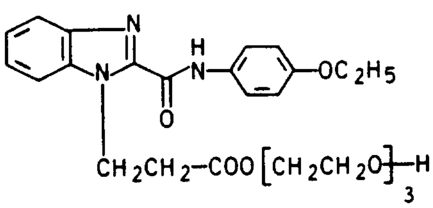

Gelbliches Öl
Analyse: $C_{25}$ $H_{21}$ $N_3$ $O_7$ (485,5)
UV($CH_2Cl_2$): $\lambda_{max}$ ($\epsilon \cdot 10^{-3}$) = 312 nm (22,4)

Beispiel 34

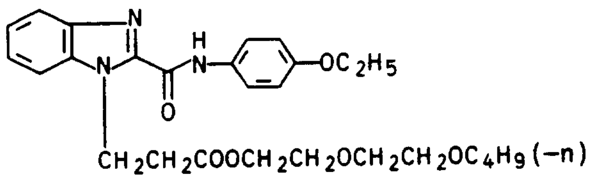

Gelbliches Öl
Analyse: $C_{27} H_{35} N_3 O_6$ (497,6)
UV($CH_2Cl_2$): $\lambda_{max}$ ($\epsilon \cdot 10^{-3}$) = 312 nm (21,6)

Beispiel 35

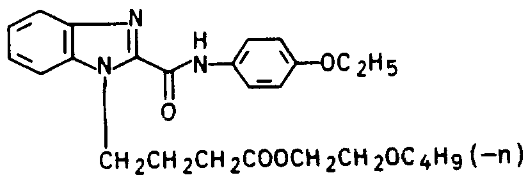

Gelbliches Öl

| Analyse: $C_{26} H_{33} N_3 O_5$ (467,5) | | | | |
|---|---|---|---|---|
| Ber.: | C 66,81 | H 7,07 | N 8,99 | O 17,13 |
| Gef.: | C 66,4 | H 7,3 | N 9,0 | O 17,5 |

Beispiel 36

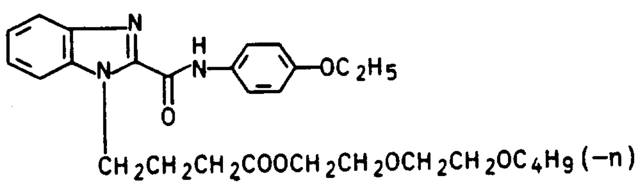

Gelbes Öl.
Analyse: $C_{28} H_{37} N_3 O_6$ (511,6)
UV($CH_2Cl_2$): $\lambda_{max}$ ($\epsilon \cdot 10^{-3}$) = 312 nm (23,0)

Beispiel 37 - 45

Durch Umesterung mit Polyethylenglykolen analog Beispiel 31 erhält man die folgenden Polyethylenglykolester der entsprechenden Benzimidazol-1-carbonsäuren,die in Form hellgelber Harze oder Öle nach der in Beispiel 31 beschriebenen Methode isoliert werden. Nach $^1$H-NMR-Analyse liegen diese Verbindungen fast ausschließlich als Monoester der folgenden Formel vor.

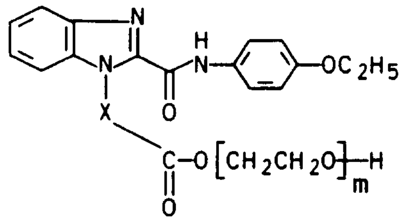

| Bsp. | X | mittleres Molekulargewicht der Polyglykols | | gefundene Analysenwerte | | | |
|---|---|---|---|---|---|---|---|
| | | | | C | H | N | O |
| 37 | $CH_2$ | 200 | $\overline{m}$ = 4,5 | 60,8 | 6,7 | 8,2 | 24,9 |
| 38 | $(CH_2)_2$ | 200 | | 62,1 | 6,6 | 8,5 | 23,5 |
| 39 | $(CH_2)_3$ | 200 | | 61,7 | 6,8 | 7,8 | 23,9 |
| 40 | $CH_2$ | 300 | $\overline{m}$ = 6,8 | 60,0 | 6,8 | 7,3 | 26,2 |
| 41 | $(CH_2)_2$ | 300 | | 60,7 | 6,9 | 7,2 | 25,1 |
| 42 | $(CH_2)_3$ | 300 | | 61,0 | 7,2 | 6,9 | 25,2 |
| 43 | $CH_2$ | 400 | $\overline{m}$ = 9,1 | 59,1 | 7,1 | 6,2 | 27,6 |
| 44 | $(CH_2)_2$ | 400 | | 59,9 | 7,3 | 6,3 | 26,9 |
| 45 | $(CH_2)_3$ | 400 | | 60,4 | 7,4 | 6,3 | 26,6 |

Beispiel 46

35,3 g 1-Carbomethoxymethyl-4'-ethoxy-benzimidazol-2-carbonsäureanilid (Verbindung aus Bsp. 4) werden mit 130 ml Cyclohexylamin unter Stickstoff 2,5 Stunden auf 130 °C erhitzt, wobei das entstehende Methanol abdestilliert wird. Nach dem Abkühlen wird mit 250 ml Methanol verdünnt, das ausgefallene Produkt abgesaugt und mit Methanol gewaschen. Man erhält 20,3 g eines farblosen Feststoffes der Formel

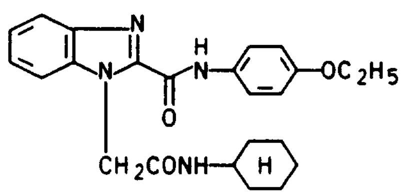

mit dem Schmp.: 239 °C.

| Analyse: $C_{24}$ $H_{28}$ $N_4$ $O_3$ (420,3) | | | | |
|---|---|---|---|---|
| Ber.:<br>Gef.: | C 68,54<br>C 68,7 | H 6,71<br>H 6,9 | N 13,33<br>N 13,2 | O 11,42<br>O 11,2 |
| UV($CH_2Cl_2$): $\lambda_{max}$) ($\epsilon \cdot 10^{-3}$) = 314 nm (19,6) | | | | |

Beispiel 47

Nach der in Beispiel 46 beschriebenen Verfahrensweise erhält man mit Benzylamin ein Produkt der Formel

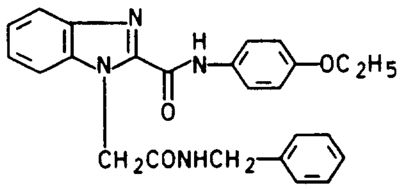

mit dem Schmp.: 198 - 200 ° C.

| Analyse: $C_{25}$ $H_{24}$ $N_4$ $O_3$ (428,5) | | | | |
|---|---|---|---|---|
| Ber.:<br>Gef.: | C 70,09<br>C 70,4 | H 5,61<br>H 5,8 | N 13,08<br>N 13,2 | O 11,22<br>O 10,8 |

Beispiel 48

Verwendet man nach Beispiel 46 Anilin statt Cyclohexylamin, so erhält man das 1-Carboxyanilido-methyl-benzimidazol der Formel

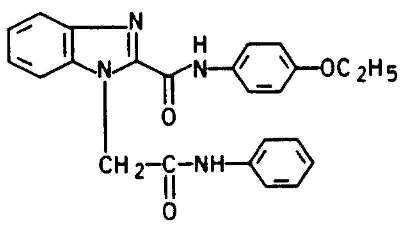

mit dem Schmp.: 246 - 248 ° C.

| Analyse: $C_{24}$ $H_{22}$ $N_4$ $O_3$ (414,3) | | | | |
|---|---|---|---|---|
| Ber.:<br>Gef.: | C 69,57<br>C 69,6 | H 5,32<br>H 5,4 | N 13,52<br>N 13,4 | O 11,59<br>O 11,4 |

Beispiel 49

35,3 g 1-Carbomethoxymethyl-4'-ethoxy-benzimidazol-2-carbonsäureanilid (Verbindung aus Bsp. 4) werden in 70 ml 3-(2-Ethylhexoxy)-propylamin 4,5 Stunden bei einer Temperatur von 135 bis 140 ° C gerührt. Die Reaktionsmischung wird nach dem Abkühlen auf ca. 80 ° C in 1000 ml Methanol gegossen. Durch langsame Zugabe von 600 ml Eiswasser wird das Produkt ausgefällt, abgesaugt und mit Wasser gewaschen. Der erhaltene Rückstand wird nochmals in 500 ml Methanol in der Hitze gelöst, mit Aktivkohle behandelt, und wieder durch Zugabe von 600 ml Eiswasser gefällt. Nach dem Absaugen, Waschen und Trocknen resultieren 43,0 g farbloser Feststoff mit der Formel

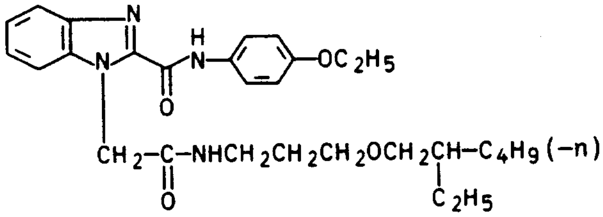

und dem Schmp.: 140 - 141 °C.

| Analyse: $C_{29}H_{40}N_4O_4$ (508,7) | | | | |
|---|---|---|---|---|
| Ber.: | C 68,50 | H 7,88 | N 11.02 | O 12,6 |
| Gef.: | C 68,2 | H 8,0 | N 10,9 | O 12,2 |

Beispiel 50

Durch Umsetzen von 1-Carbomethoxyethyl-4'-ethoxy-benzimidazol-2-carbonsäureanilid (aus Bsp. 5) mit 3-(2-Ethylhexoxy)-propylamin analog Beispiel 49 erhält man die Verbindung der Formel

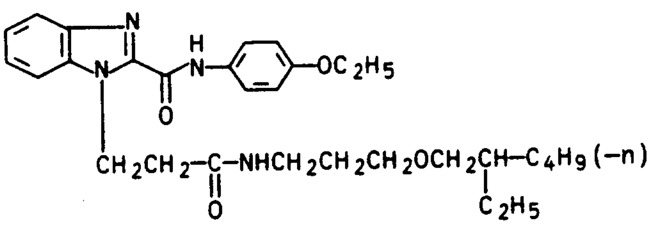

mit dem Schmp.: 118 - 119 °C.

| Analyse: $C_{30}H_{42}N_4O_4$ (522,7) | | | | |
|---|---|---|---|---|
| Ber.: | C 68, 96 | H 8,05 | N 10,73 | O 12,26 |
| Gef.: | C 69,1 | H 8,2 | N 10,6 | O 12,3 |

Beispiel 51

Analog Beispiel 49 und 50 wird aus 1-Carbomethoxypropyl-4'-ethoxybenzimidazol-2-carbonsäureanilid (aus Bsp. 6) die Verbindung der Formel

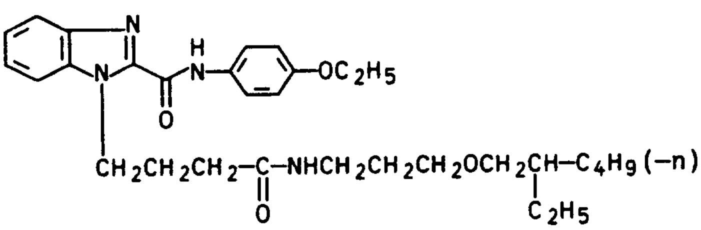

mit dem Schmp.: 108 - 110 °C erhalten.

| Analyse: $C_{31}H_{44}N_4O_4$ (536,8) | | | | |
|---|---|---|---|---|
| Ber.: | C 69,40 | H 8,22 | N 10,44 | O 11,94 |
| Gef.: | C 69,2 | H 8,4 | N 10,3 | O 11,9 |

Beispiel 52

57,2 g 1-Carbomethoxypropyl-4'-ethoxybenzimidazol-2-carbonsäureanilid (Verbindung aus Bsp. 6) und 12 g p-Toluolsulfonsäure werden in 230 ml Iso-Decanol 3 Stunden bei 140 °C gerührt. Die heiße

Reaktionsmischung wird in 1 l Eiswasser gegossen und anschließend mit 500 ml Essigsäureethylester extrahiert. Die organische Phase wäscht man zweimal mit 500 ml Wasser und reinigt sie dann mit Aktivkohle und Bleicherde. Das Lösungsmittel wird im Vakuum abdestilliert und man erhält 69 g eines schwach gelben Öles, das dem Produkt mit der Formel

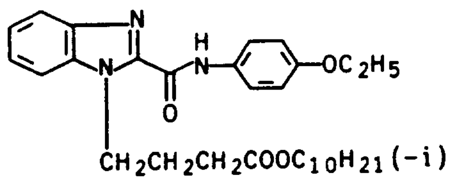

entspricht.
$C_{30}H_{41}N_3O_4$ (509,4)
UV ($CH_2Cl_2$): $\lambda_{max.}$ ($\epsilon \cdot 10^{-3}$) = 313 nm (20,8).

Beispiel 53

Zu einer Suspension aus 73,4 g 1-(3-Carboxypropoxypropyl)4'-ethoxy-benzimidazol-2-carbonsäureanilid (Verbindung aus Bsp. 3) und 400 ml n-Butyltriglykol werden bei -5 bis 0 °C 26,2 g Thionylchlorid zugetropft. Die Reaktionsmischung wird 0,5 Stunden bei Raumtemperatur und 2,5 Stunden bei 80 °C gerührt und anschließend auf 1,5 l Eiswasser gegeben. Man stellt mit $NaHCO_3$ auf pH 7 - 8 und extrahiert 3mal mit 500 ml Essigsäureethylester. Die vereinigten organischen Phasen werden mit 500 ml Wasser gewaschen, über $Na_2SO_4$ getrocknet und mittels Aktivkohle und Bleicherde gereinigt. Nach dem Einengen im Vakuum verbleiben 105 g eines gelblichen Öles, das dem Produkt mit der Formel

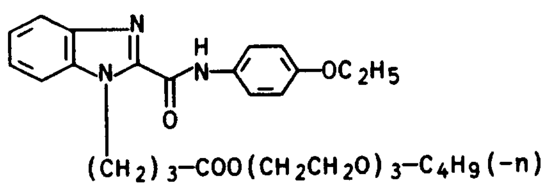

entspricht.
$C_{30}H_{41}N_3O_4$ (555,3)
UV ($CH_2Cl_2$): $\lambda_{max.}$ ($\epsilon \cdot 10^{-3}$) = 313 nm (19,1).

Beispiel 54

38,1 g des Produkt aus Beispiel 6 wurden in 100 ml n-Octylamin 7,5 h auf 140 bis 145 °C erhitzt. Nach Abdestillieren von ca. 20 ml n-Octylamin wurde die Reaktionsmischung mit 700 ml Ligroin versetzt, der ausgefallene Niederschlag abgesaugt, mit Ligroin gewaschen und getrocknet. Man erhielt 41,4 g der Verbindung der Formel

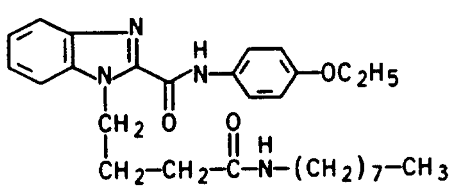

als farblosen Feststoff von Schmp. 137 bis 138 °C .

| Analyse: $C_{28}H_{38}N_4O_3$ (478,3) | | | | |
|---|---|---|---|---|
| Ber.: | C 70,25 | H 8,01 | N 11,71 | O 10,03 |
| Gef.: | C 70,1 | H 8,3 | N 11,8 | O 10,2 |

Beispiel 55

30,5 g des Produktes aus Beispiel 6 wurden in 100 ml Oleylamin 13 h auf 140 bis 145 °C erhitzt. Nach Abkühlen auf 70 °C wurde die Reaktion in 800 ml Methanol gegossen und 1 h gerüht. Der ausgefallene Niederschlag wurde abgesaugt, mit Methanol gewaschen und bei 50 °C in Vakuum getrocknet. Man erhielt 25,5 g Verbindung der Formel

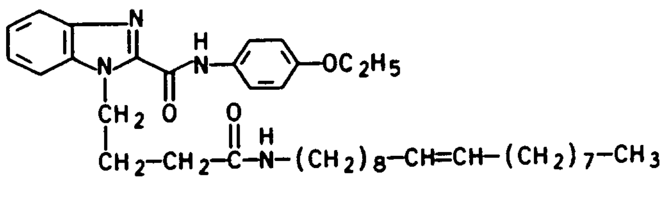

als farblosen Feststoff vom Schmp. 114 bis 116 °C .

Beispiel 57

In eine Lösung von 18,4 g des Produktes aus Beispiel 3 und 14,2 g Di-n-butylamin in 180 ml Toluol wurden bei 10 bis 15 °C Thionylchlorid zugetropft. Nach 1,5 h bei 40 °C und 6 h bei 80 °C wurde eine Mischung aus 300 ml Eiswasser und 100 ml 10 %iger Salzsäure gegossen. Die abgetrennte organische Phase wurde mit 300 ml 10 %iger Salzsäure und anschließend mit gesättigter Natriumhydrogencarbonatlösung ausgeschüttelt. Die organische Phase wurde 2 mal mit je 7,5 g Aktivkohle und 7,5 g Bleicherde 1 h lang ausgekocht. Nach dem Filtrieren wurde das Lösungsmittel im Wasserstrahlvakuum abdestilliert und der Rückstand aus 450 ml Ligroin umkristallisiert. Man erhielt nach Absaugen und Trocknen 13,3 g der Verbindung der Formel

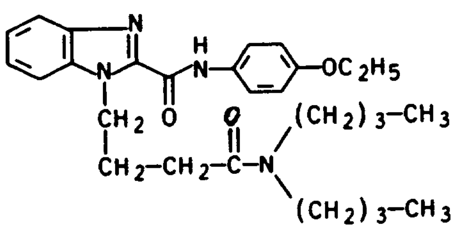

als farblosen Feststoff von Schmp. 78 bis 80 °C.

Beispiele 58 bis 64

Analog Beispiel 57 erhält man durch Umsetzung des Produkts aus Beispiel 3 mit Aminen und Thionylchlorid in Toluol die Amide der folgenden Formel:

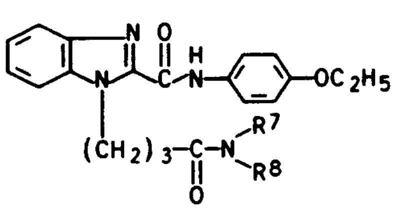

| Bsp. | 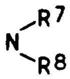 | Fp.(°C) | Analyse |
|---|---|---|---|
| 58 | $N-[CH_2-CH(C_2H_5)-(CH_3)_3-CH_3]_2$ | Öl | ber.C 73,2 H 9,2 N 9,5 O 8,1<br>gef.C 72,5 H 9,1 N 9,1 O 8,9 |
| 59 | 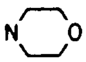 | 115-117 | ber.C 66,0 H 6,4 N 12,8 O 14,7<br>gef.C 65,5 H 6,7 N 12,4 O 15,5 |
| 60 | 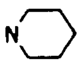 | 48- 52 | ber.C 69,1 H 7,0 N 12,9 O 11,0<br>gef.C 68,5 H 7,3 N 12,4 O 11,7 |
| 61 | 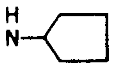 | 185-186 | ber.C 69,1 H 7,0 N 12,9 O 11,0<br>gef.C 69,1 H 7,2 N 13,0 O 11,1 |
| 62 | $\overset{H}{N}-CH_2-CH(CH_3)_2$ | 181 | |
| 63 | $\overset{H}{N}-CH(CH_3)-CH_2-CH_3$ | 160 | |
| 64 | $\overset{H}{N}-(CH_2)_3-CH_3$ | 149 | |

B) Anwendungsbeispiele

Anwendungsbeispiel 1 (Lichtstabilisierende Wirkung in Polyurethan)

Herstellung der Belichtungsproben aus Polyurethan

Eine Polyolkomponente der Zusammensetzung 41,9 Teile eines Polyetherols der OH-Zahl von 29,0 das durch Addition von Propylenoxid und Ethylenoxid an Propylenglykol erhalten wurde und ungefähr 84 % primäre Hydroxylgruppen besitzt und 42,5 Teile eines Polyetherols der OH-Zahl von 27,0, das durch Addition von Propylenoxid und Ethylenoxid an Trimethylolpropan erhalten wurde und ungeführ 88 % primäre Hydroxylgruppen besitzt und 8,1 Teile 1,4-Butandiol und 1,724 Teile 25 %ige Lösung von Diazabicyclooctan in 1,4-Butandiol und 0,016 Teile Dibutylzinndilaurat und 0,1 Teile des Siliconstabilisators OS 710 der Firma Bayer und 5,49 Teile Frigen 11 und 0.17 Teile Wasser wurde mit den angegebenen Stabilisatoren versetzt und im Mischungsverhältnis 100 : 48,5 mit einem 23,0 %-isocyanatgruppenhaltigen Prepolymeren bei 25 °C Komponenten- und Werkzeugtemperatur zu Prüfplatten verschäumt. Das NCO-Prepolymer wurde hergestellt aus 87,17 Teilen 4,4'-Diphenylmethandiisocyanat und 4,83 Teilen eines Polyetherols mit der OH-Zahl von 250, das durch Addition von Propylenoxid an Propylenglykol erhalten wurde und 8,0 Teile Dipropylenglykol.

Die Proben wurden im Xenotest® 450 belichtet und an den Proben der Yellowness Index (YI) gemäß ASTM D 1925 bestimmt. Die Ergebnisse sind in der Tabelle 2 zusammengestellt.

Tabelle 2

| Stabilisator | | Konzentration [%] | YI nach ASTM D 1925 0 H | 96 h |
|---|---|---|---|---|
| 2.1 | 0 (Kontrolle) | — | 1,4 | 31,7 |
| 2.2 | 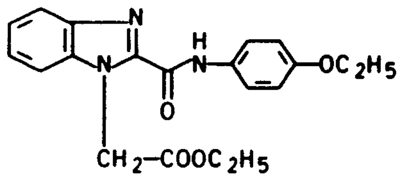 (Bsp. 9) | 1,0 | 1,9 | 15,6 |
| 2.3 | 2-(2'-Hydroxy-5'-methyl-phenyl)-benztriazol | 0,5 | | |
| | + A*) | 0,5 | 2,7 | 23,2 |
| | + Triethylenglykol-bis-3--(3-t-butyl-4-hydroxy-5--methylphenyl)-propionat | 0,25 | | |

*)A : 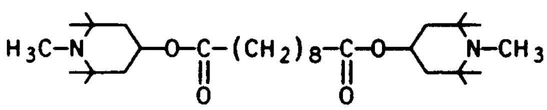

Anwendungsbeispiel 2

Die folgenden in Tabelle 3 aufgeführten Proben wurden analog Anwendungsbeispiel 1 präpariert und geprüft.

Tabelle 3

| Stabilisator | | Konzentration [%] | YI nach ASTM D 1925 | |
|---|---|---|---|---|
| | | | 0 H | 96 h |
| 3.1 | 0 (Kontrolle) | - | 5,0 | 32,5 |
| 3.2 | Verbind. aus Bsp. 27<br>+ A<br>+ B**) | 0,5<br>0,5<br>0,25 | 4,2 | 21,1 |
| 3.3 | Verbind. aus Bsp. 19<br>+ A<br>+ B**) | 0,5<br>0,5<br>0,25 | 5,9 | 20,6 |
| 3.4 | Verbind. aus Bsp. 28<br>+ A<br>+ B**) | 0,5<br>0,5<br>0,25 | 5,0 | 22,8 |
| 3.5 | verbind. aus Bsp. 10<br>+ A<br>+ B | 0,5<br>0,5<br>0,25 | 3,2 | 16,2 |

**) B : Gemisch aus 1 Gew.-Teil α-Tocopherol und 10 Gew.-Teilen Tris(nonyl-phenyl)phosphit

Anwendungsbeispiel 3

Nach der im Anwendungsbeispiel 1 beschriebenen Methode wurden die in Tabelle 4 aufgeführten Proben hergestellt und belichtet. Zur Stabilisierung wurden jeweils Gemische aus 0,5 % UV-Absorber, 0,5 % sterisch gehindertem Amin (Verbindung A aus Anwendungsbeispiel 1) und 0,25 % Antioxidans (Triethylenglykol-bis-3-(3-t-butyl-4-hydroxy-5-methylphenyl)-propionat) verwendet.

Tabelle 4

| UV-Absorber | YI nach ASTM D 1925 nach 0 h | YI nach ASTM D 1925 nach 96 h |
|---|---|---|
| 4.1 unstabilisierte Probe | 9,6 | 55,5 |
| 4.2 Verbind. aus Bsp. 37 | 5,7 | 22,4 |
| 4.3 Verbind. aus Bsp. 31 | 6,3 | 21,9 |
| 4.4 Verbind. aus Bsp. 39 | 5,5 | 18,3 |
| 4.5 Verbind. aus Bsp. 40 | 6,8 | 22,4 |
| 4.6 Verbind. aus Bsp. 42 | 5,0 | 21,7 |
| 4.7 Verbind. aus Bsp. 44 | 5,6 | 21,5 |
| 4.8 C***) | 5,7 | 21,7 |

***)C: Umsetzungsprodukt von

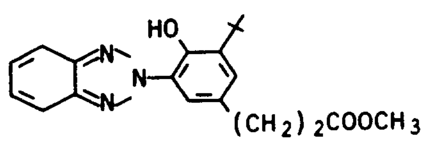

mit einem Polyethylenglykol der mittleren Molekülmasse von 300.

Anwendungsbeispiel 4

Flüchtigkeit von Stabilisatoren im Klarlack eines Zweischicht-Metallicsystems

Jeweils 0,4 g des in Tabelle 5 angegebenen Stabilisators werden in 55,6 g spritzfertigem Klarlack, bestehend aus 1500 Teilen Acrylat-Einbrennlack und 167 Teilen Xylol, bis zur vollständigen Lösung eingerührt.

Die fertige Lacklösung wird auf ein Quarzglas aufgerakelt (Schichtdicke ca. 40 µm) und 5 Stunden bei 140 °C eingebrannt. Zur Bestimmung des Verlustes an UV-Absorber während des Einbrennvorganges werden vor und nach dem Einbrennen Absorptionsspektren aufgenommen und der Verlust aus der Abnahme der optischen Dichte im Absorptionsmaximum bestimmt.

Tabelle 5

| Stabilisator | | Prozentualer Verlust nach 5 h bei 140 °C |
|---|---|---|
| 5.1 | Verbindung aus Bsp. 6 | 7 |
| 5.2 | Verbindung aus Bsp. 20 | 5 |
| 5.3 | Verbindung aus Bsp. 25 | 6,3 |
| 5.4 | Verbindung aus Bsp. 32 | 2,7 |
| 5.5 | Verbindung aus Bsp. 34 | 3,7 |
| 5.6 | Verbindung aus Bsp. 36 | 5 |
| 5.7 | Verbindung aus Bsp. 39 | 4 |
| 5.8 | Verbindung aus Bsp. 40 | 4 |
| 5.9 | Verbindung aus Bsp. 42 | 4 |
| 5.10 | Verbindung aus Bsp. 51 | 6 |

## Patentansprüche

**1.** Verbindungen der Formel I

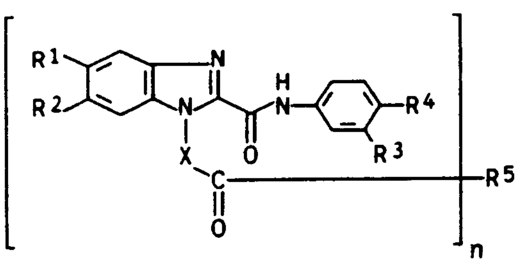 (I),

in der

- $R^1$ und $R^2$ unabhängig voneinander für H, Cl, $C_1$- bis $C_4$-Alkyl, $C_1$- bis $C_4$-Alkoxy, für gegebenenfalls durch $C_1$- bis $C_4$-Alkyl oder $C_1$- bis $C_4$-Alkoxy substituiertes Phenyl oder $C_7$- bis $C_9$-Phenalkyl,
- $R^3$ und $R^4$ unabhängig voneinander für H, $C_1$- bis $C_{18}$-Alkyl, durch -O- ein- oder mehrfach unterbrochenes $C_3$- bis $C_{18}$-Alkyl, $C_1$- bis $C_{18}$-Alkoxy, durch -O- ein- oder mehrfach unterbrochenes $C_4$- bis $C_{18}$-Alkoxy, gegebenenfalls durch $C_1$- bis $C_4$-Alkyl oder durch $C_1$- bis $C_4$-Alkoxy substituiertes Phenyl, $C_7$- bis $C_{15}$-Phenylalkyl, Phenoxy, $C_7$- bis $C_{15}$-Phenylalkyloxy, für $C_1$- bis $C_4$-Hydroxyalkyl, $C_1$- bis $C_4$-Hydroxyalkoxy, $C_1$- bis $C_{12}$-Alkylcarbonylamino, $C_1$- bis $C_{12}$-Alkanoyloxy, Benzoylamino oder Benzoyloxy oder $R^3$ und $R^4$ zusammen für Methylendioxy oder Ethylendioxy,
- X für $C_1$- bis $C_5$-Alkylen,
- n für 1 oder 2 und
- $R^5$

a) - wenn n = 1 ist - für
Cl, $-OR^6$ oder

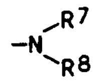

oder
b) - wenn n = 2 ist - für
einen zweiwertigen Rest der Formeln
$-O-R^{12}-O-$ oder

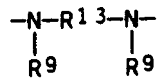

stehen,

worin

$R^6$ H, gegebenenfalls durch 1 bis 3 OH-Gruppen oder durch $-O-COR^9$ substituiertes $C_1$-$C_{18}$-Alkyl, durch -O-, $-NR^9$- ein- oder mehrfach unterbrochenes $C_3$-$C_{18}$-Alkyl, das gegebenenfalls durch -OH substituiert ist, gegebenenfalls durch OH substituiertes $C_5$-$C_{12}$-Cycloalkyl, gegebenenfalls durch OH substituiertes $C_2$-$C_{18}$-Alkenyl, $C_7$-$C_{15}$-Phenylalkyl, Phenoxyethyl,

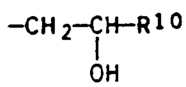 oder 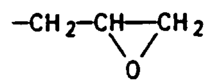

$R^7$ und $R^8$ unabhängig voneinander H, $C_1$-$C_{18}$-Alkyl, durch O, $-NR^9$-ein- oder mehrfach unterbrochenes $C_3$-$C_{18}$-Alkyl, $C_5$-$C_{12}$-Cycloalkyl, Phenyl, Naphthyl, $C_3$-$C_{18}$-Alkenyl, $C_7$-$C_{15}$-Phenylalkyl, $C_2$-$C_4$-Hydroxyalkyl oder

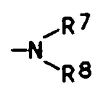

Pyrrolidinyl, Piperidinyl, Piperazinyl oder Morpholinyl,

$R^9$ H, $C_1$-$C_{18}$-Alkyl, $C_5$-$C_{12}$-Cycloalkyl, $C_3$-$C_8$-Alkenyl, Decenyl, Oleyl, Phenyl, Naphthyl oder $C_7$-$C_{18}$-Phenylalkyl,

$R^{10}$ H, $C_1$- bis $C_{18}$-Alkyl, $C_7$-$C_{18}$-Phenylalkyl, gegebenenfalls substituiertes Phenyl oder $-CH_2OR^{11}$,

$R^{11}$ $C_1$- bis $C_{18}$-Alkyl, $C_3$- bis $C_{18}$-Alkenyl, $C_5$- bis $C_{12}$-Cycloalkyl, Phenyl, $C_7$- bis $C_{15}$-Phenylalkyl,

$R^{12}$ $C_2$- bis $C_8$-Alkylen, $C_4$- bis $C_8$-Alkenylen, Cyclohexylen, durch -O-ein-oder mehrfach unterbrochenes $C_4$- bis $C_{18}$-Alkylen,

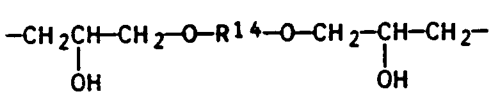 oder 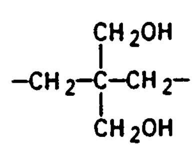 ,

$R^{13}$ gegebenenfalls durch -O- ein- oder mehrfach unterbrochenes $C_2$-bis $C_{12}$-Alkylen,

Cyclohexylen,

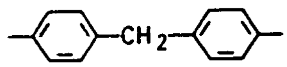 oder 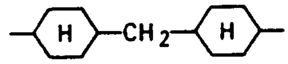 oder

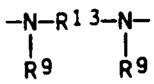

einen 2-wertigen Piperazinrest, und

$R^{14}$ $C_2$- bis $C_8$-Alkylen, durch -O- ein- oder mehrfach unterbrochenes $C_4$-bis $C_{18}$-Alkylen, 1,3- oder 1,4-Cyclohexylen, 1,3- oder 1,4-Phenylen,

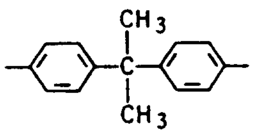 oder 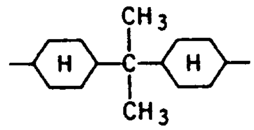 bedeuten.

**2.** Verbindungen nach Anspruch 1, dadurch gekennzeichnet, daß $R^1$ und $R^2$ unabhängig voneinander für H, Cl, $C_1$- bis $C_4$-Alkyl, $C_1$- bis $C_4$-Alkoxy, $C_7$- bis $C_9$-Phenylalkyl,

$R^3$ und $R^4$ unabhängig voneinander für H, $C_1$- bis $C_{12}$-Alkyl, $C_1$- bis $C_{12}$-Alkoxy, durch -O- ein- oder mehrfach unterbrochenes $C_3$- bis $C_{10}$-Alkyl oder $C_3$- bis $C_{10}$-Alkoxy für $C_7$- bis $C_9$-Phenylalkyl, $C_7$- bis $C_9$-Phenylalkoxy, Phenoxy, $C_1$- bis $C_{12}$-Alkylcarbonyloxy, Benzoyloxy, $C_1$- bis $C_{12}$-Alkylcarbonylamino, Benzoylamino oder $R^3$ und $R^4$ zusammen für Methlyendioxy oder Ethylendioxy und

X für $C_1$ bis $C_5$-Alkylen stehen.

**3.** Verbindungen gemäß Anspruch 2, dadurch gekennzeichnet, daß

n für 1 und

$R^5$ für

-$OR^6$ oder

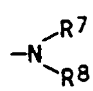

stehen, wobei

$R^6$ H, $C_1$- bis $C_{18}$-Alkyl, das gegebenenfalls durch -OH oder -$OCOR^9$ substituiert ist, durch -O- ein- oder mehrfach unterbrochenes $C_3$- bis $C_{18}$-Alkyl, das gegebenenfalls durch -OH substituiert ist, gegebenenfalls durch -OH substituiertes $C_5$- bis $C_{12}$-Cycloalkyl oder $C_2$- bis $C_{18}$-Alkenyl, $C_7$- bis $C_{15}$-Phenylalkyl, Phenoxyethyl,

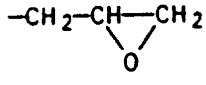 oder 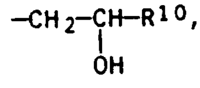

$R^7$ und $R^8$ unabhängig voneinander für H, $C_1$- bis $C_{12}$-Alkyl, durch -O-, -$NR^9$- ein- oder mehrfach unterbrochenes $C_3$- bis $C_{18}$-Alkyl, $C_5$- bis $C_{12}$-Cycloalkyl, Phenyl, $C_7$- bis $C_{15}$-Phenylalkyl oder $C_2$- bis $C_4$-Hydroxyalkyl, oder

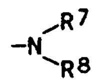

einen Pyrrolidin-, Piperidin-, Piperazin- oder Morpholinrest,

- $R^9$ H, $C_1$- bis $C_4$-Alkyl, $C_5$- bis $C_{12}$-Cycloalkyl, Phenyl oder Benzyl,
- $R^{10}$ H, $C_1$- bis $C_{12}$-Alkyl oder-$CH_2OR^{11}$ und
- $R^{11}$ $C_1$- bis $C_{12}$-Alkyl, $C_5$- bis $C_{10}$-Cycloalkyl, Phenyl oder $C_7$- bis $C_9$-Phenylalkyl bedeuten.

**4.** Verbindungen nach Anspruch 2, dadurch gekennzeichnet, daß

- n für 2 und
- $R^5$ für einen zweiwertigen Rest der Formeln -O-$R^{12}$-O- oder

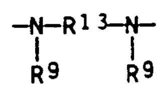

stehen, worin

- $R^9$ H, $C_1$- bis $C_{18}$-Alkyl, Cyclohexyl,
- $R^{12}$ $C_2$- bis $C_8$-Alkylen, durch -O- ein- oder mehrfach unterbrochenes $C_4$- bis $C_{18}$-Alkylen oder

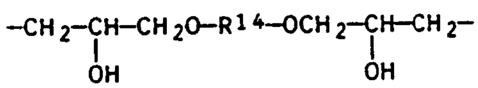 ,

- $R^{13}$ gegebenenfalls durch -O- ein oder mehrfach unterbrochenes $C_2$- bis $C_{12}$-Alkylen, Cyclohexylen,

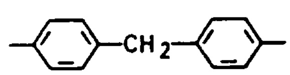 oder 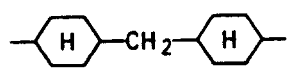

und

- $R^{14}$ $C_2$- bis $C_8$-Alkylen, durch -O- ein- oder mehrfach unterbrochenes $C_4$- bis $C_{18}$-Alkylen, 1,3- oder 1,4-Cyclohexylen, 1,3- oder 1,4-Phenylen,

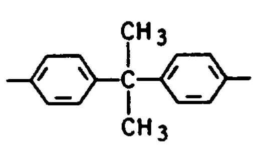 oder 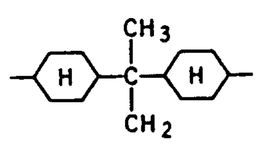 bedeuten.

**5.** Verbindungen nach Anspruch 1, dadurch gekennzeichnet, daß

- $R^1$ und $R^2$ unabhängig voneinander für H, Cl, Methyl, Ethyl, Methoxy, Ethoxy, Benzyl, Methylbenzyl oder $\alpha,\alpha$-Dimethylbenzyl,
- $R^3$ und $R^4$ unabhängig voneinander für H, $C_1$- bis $C_6$-Alkyl, $C_1$- bis $C_8$-Alkoxy, $C_7$- bis $C_9$-Phenylalkyl, $C_7$- bis $C_9$-Phenylalkoxy, Phenoxy, Benzoyloxy, $C_1$- bis $C_6$-Alkanoyloxy, $C_1$- bis $C_6$-Alkanoylamino oder Benzoylamino oder $R^3$ und $R^4$ zusammen für Methylendioxy oder Ethylendioxy,
- X für $C_1$- bis $C_5$-Alkylen,
- n für 1 und

$R^5$ für $-OR^6$ oder

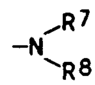

stehen, worin

$R^6$ H, $C_1$- bis $C_{18}$-Alkyl, durch -O- einfach oder mehrfach unterbrochenes $C_3$- bis $C_{18}$-Alkyl, das gegebenenfalls durch -OH substituiert ist, gegebenenfalls durch -OH substituiertes $C_5$- bis $C_{12}$-Cycloalkyl, $C_2$- bis $C_{18}$-Alkenyl, $C_7$- bis $C_9$-Phenylalkyl, Phenoxyethyl,

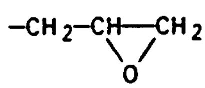 oder 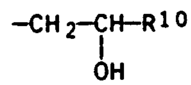 ,

einer der Reste

$R^7$ und $R^8$ H und der andere Rest $R^7$ und $R^8$ $C_1$- bis $C_{12}$-Alkyl, durch -O-, $-NR^9$- ein- oder mehrfach unterbrochenes $C_3$- bis $C_{18}$-Alkyl, $C_5$- bis $C_{12}$-Cycloalkyl, Phenyl, $C_9$-Phenylalkyl oder $C_2$- bis $C_4$-Hydroxyalkyl, $C_2$- bis $C_4$-Hydroxyalkyl,

$R^9$ H oder $C_1$- bis $C_4$-Alkyl,

$R^{10}$ H, $C_1$- bis $C_{12}$-Alkyl oder $-CH_2OR^{11}$ und

$R^{11}$ $C_1$- bis $C_{12}$-Alkyl, $C_3$- bis $C_4$-Alkenyl, Cyclohexyl, Phenyl oder $C_7$- bis $C_9$-Phenylalkyl bedeuten.

**6.** Verbindungen nach Anspruch 1, dadurch gekennzeichnet, daß

$R^1$ und $R^2$ unabhängig voneinander für H, Cl, Methyl, Ethyl, Methoxy, Ethoxy, Benzyl, Methylbenzyl oder $\alpha,\alpha$-Dimethylbenzyl,

$R^3$ und $R^4$ unabhängig voneinander für H, $C_1$- bis $C_6$-Alkyl, $C_1$- bis $C_8$-Alkoxy, $C_7$- bis $C_9$-Phenylalkyl, $C_7$- bis $C_9$-Phenylalkoxy, Phenoxy, Benzoyloxy, $C_1$- bis $C_6$-Alkanoyloxy, $C_1$- bis $C_6$-Alkanoylamino oder Benzoylamino oder $R^3$ und $R^4$ zusammen für Methylendioxy oder Ethylendioxy,

X für $C_1$- bis $C_5$-Alkylen,

n für 2 und

$R^5$ für einen zweiwertigen Rest der Formeln

$-O-R^{12}-O-$ oder

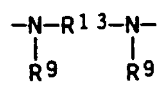

stehen, worin

$R^9$ H, $C_1$- bis $C_6$-Alkyl oder Cyclohexyl,

$R^{12}$ $C_2$- bis $C_6$-Alkylen oder durch -O- ein- oder mehrfach unterbrochenes $C_4$- bis $C_{18}$-Alkylen und

$R^{13}$ gegebenenfalls durch -O- ein- oder mehrfach unterbrochenes $C_2$-bis $C_{12}$-Alkylen oder Cyclohexylen bedeuten.

**7.** Verwendung der Verbindungen gemäß Anspruch 1, 2, 3, 4, 5 oder 6 als Lichtschutzmittel für organische Materialien.

**8.** Stabilisiertes organisches Material, enthaltend 0,05 bis 10 Gew.-% - bezogen auf das organische Material - einer oder mehrerer Verbindungen gemäß Anspruch 1, 2, 3, 4, 5 oder 6.

**9.** Stabilisiertes organisches Material nach Anspruch 8, dadurch gekennzeichnet, daß das Material ein synthetisches Polymer ist.

**10.** Stabilisiertes organisches Material gemäß Anspruch 9, dadurch gekennzeichnet, daß das Polymer ein Polyester, Polyamid, heißvernetzbares Acrylharz, thermoplastisches Acrylharz, MF- oder UF-Harz, ABS oder ein Polyurethan ist.

**11.** Lack, enthaltend stabilisiertes organisches Material gemäß Anspruch 10.

## Claims

**1.** A compound of the formula I

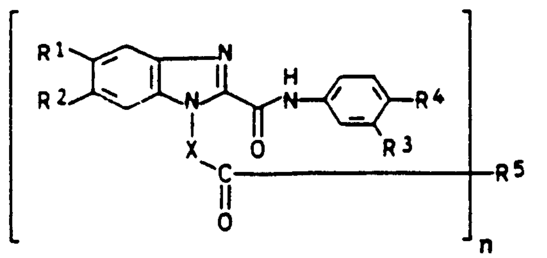

(I),

where

| | |
|---|---|
| $R^1$ and $R^2$ | are each independently of the other H, Cl, $C_1$-$C_4$-alkyl, $C_1$-$C_4$-alkoxy, unsubstituted or $C_1$-$C_4$-alkyl- or $C_1$-$C_4$-alkoxy-substituted phenyl or $C_7$-$C_9$-phenalkyl, |
| $R^3$ and $R^4$ | are each independently of the other H, $C_1$-$C_{18}$-alkyl, $C_3$-$C_{18}$-alkyl which is interrupted one or more times by -O-, $C_1$-$C_{18}$-alkoX, $C_1$-$C_{18}$-alkoxy which is interrupted one or more times by -O-, unsubstituted or $C_1$-$C_4$-alkyl- or $C_1$-$C_4$-alkoxy-substituted phenyl, $C_7$-$C_{15}$-phenylalkyl, phenoxy, $C_7$-$C_{15}$-phenylalkyloxy, $C_1$-$C_4$-hydroxyalkyl, $C_1$-$C_4$-hydroxyalkoxy, $C_1$-$C_{12}$-alkylcarbonylamino, $C_1$-$C_{12}$-alkanoyloxy, benzoylamino or benzoyloxy or $R^3$ and $R^4$ together are methylenedioxy or ethylenedioxy, |
| X | is $C_1$-$C_5$-alkylene, |
| n | is 1 or 2 and |
| $R^5$ | is |

a) - when n is 1 - Cl, $-OR^6$ or

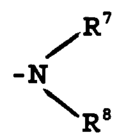

or

b) - when n is 2 - a divalent radical of the formula $-O-R^{12}-O-$ or

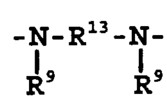

where

$R^6$ is H, unsubstituted or OH-monosubstituted, -disubstituted or -trisubstituted or $-O-COR^9$-substituted $C_1$-$C_{18}$-alkyl, $C_3$-$C_{18}$-alkyl which is interrupted one or more times by -O- or $-NR^9$- and which may in addition be OH-subsituted, unsubstituted or OH-substituted $C_5$-$C_{12}$-cycloalkyl, unsubstituted or OH-substituted $C_2$-$C_{18}$-alkenyl, $C_7$-$C_{15}$-phenylalkyl, phenoxyethyl,

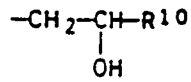 or 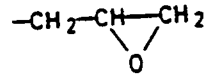

$R^7$ and $R^8$ are each independently of the other H, $C_1$-$C_{18}$-alkyl, $C_3$-$C_{18}$-alkyl which is interrupted one or more times by -O- or -$NR^9$-, $C_5$-$C_{12}$-cycloalkyl, phenyl, naphthyl, $C_3$-$C_{18}$-alkenyl, $C_7$-$C_{15}$-phenylalkyl, $C_2$-$C_4$-hydroxyalkyl or

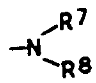

pyrrolidinyl, piperidinyl, piperazinyl or morpholinyl,

$R^9$ is H, $C_1$-$C_{18}$-alkyl, $C_5$-$C_{12}$-cycloalkyl, $C_3$-$C_8$-alkenyl, decenyl, oleyl, phenyl, naphthyl or $C_7$-$C_{18}$-phenylalkyl,

$R^{10}$ is H, $C_1$-$C_{18}$-alkyl, $C_7$-$C_{18}$-phenylalkyl, unsubstituted or substituted phenyl or -$CH_2OR^{11}$,

$R^{11}$ is $C_1$-$C_{18}$-alkyl, $C_3$-$C_{18}$-alkenyl, $C_5$-$C_2$-cycloalkyl, phenyl or $C_7$-$C_{15}$-phenylalkyl,

$R^{12}$ is $C_2$-$C_8$-alkylene, $C_4$-$C_8$-alkenylene, cyclohexylene, $C_4$-$C_{18}$-alkylene which is interrupted one or more times by -O-,

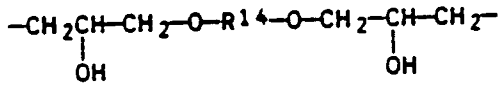 or 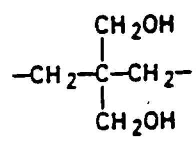,

$R^{13}$ is $C_2$-$C_{12}$-alkylene which may be interrupted one or more times by -O-, cyclohexylene,

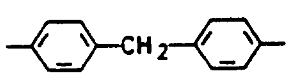 or 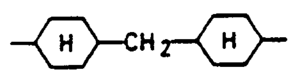 or

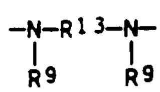,

a 2-valent piperazine radical, and

$R^{14}$ is $C_2$-$C_8$-alkylene, $C_4$-$C_{18}$-alkylene which is interrupted one or more times by -O-, 1,3- or 1,4-cyclohexylene, 1,3- or 1,4-phenylene,

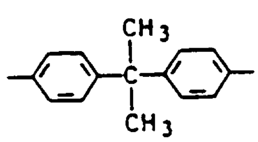 or 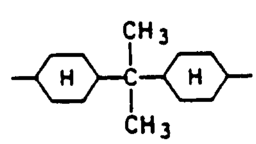

**2.** A compound as claimed in claim 1, wherein $R^1$ and $R^2$ are each independently of the other H, Cl, $C_1$-$C_4$-alkyl, $C_1$-$C_4$-alkoxy or $C_7$-$C_9$-phenylalkyl,

$R^3$ and $R^4$ are each independently of the other H, $C_1$-$C_{12}$-alkyl, $C_1$-$C_{12}$-alkoxy, $C_3$-$C_{10}$-alkyl which is interrupted one or more times by -O-, $C_3$-$C_{10}$-alkoxy which is interrupted one or more times by -O-, $C_7$-$C_9$-phenylalkyl, $C_7$-$C_9$-phenylalkoxy, phenoxy, $C_1$-$C_{12}$-alkylcarbonyloxy, benzoyloxy, $C_1$-$C_{12}$-alkylcarbonylamino or benzoylamino or $R^3$ and

$R^4$ together are methylenedioxy or ethylenedioxy, and
X is $C_1$-$C_5$-alkylene.

**3.** A compound as claimed in claim 2, wherein
n is 1 and
$R^5$ is $-OR^6$ or

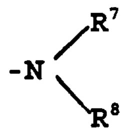

where

$R^6$ is H, $C_1$-$C_{18}$-alkyl which may be substituted by -OH or -$C_3$-$C_{18}$-alkyl which is interrupted one or more times by -O- and which may in addition be OH-substituted, unsubstituted or OH-substituted $C_5$-$C_{12}$-cycloalkyl or $C_2$-$C_{18}$-alkenyl, $C_7$-$C_{15}$-phenylalkyl, phenoxyethyl,

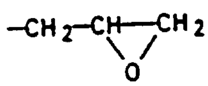 or 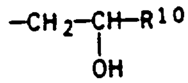,

$R^7$ and $R^8$ are each independently of the other H, $C_1$-$C_{12}$-alkyl, $C_3$-$C_{18}$-alkyl which is interrupted one or more times by -O- or $-NR^9-$, $C_5$-$C_{12}$-cycloalkyl, phenyl, $C_7$-$C_{15}$-phenylalkyl or $C_2$-$C_4$-hydroxyalkyl or

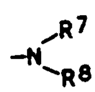

a pyrrolidine, piperidine, piperazine or morpholine radical,
$R^9$ is H, $C_1$-$C_4$-alkyl, $C_5$-$C_{12}$-cycloalkyl, phenyl or benzyl,
$R^{10}$ is H, $C_1$-$C_{12}$-alkyl or $-CH_2OR^{11}$ and
$R^{11}$ is $C_1$-$C_{12}$-alkyl, $C_5$-$C_{10}$-cycloalkyl, phenyl or $C_7$-$C_9$-phenylalkyl.

**4.** A compound as claimed in claim 2, wherein
n is 2 and
$R^5$ is a divalent radical of the formula
$-0-R^{12}-O-$ or

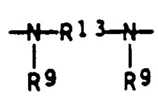

where
$R^9$ is H, $C_1$-$C_{18}$-alkyl or cyclohexyl,
$R^{12}$ is $C_2$-$C_8$-alkylene, $C_4$-$C_{18}$-alkylene which is interrupted one or more times by -O- or

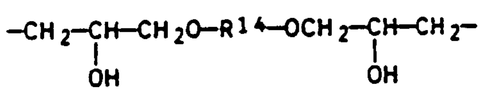,

$R^{13}$ is $C_2$-$C_{12}$-alkylene which may be inter-rupted one or more times by -O- or cyclohexylene,

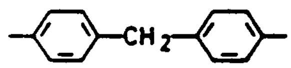 or 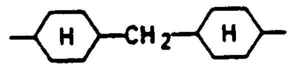

and

$R^{14}$ is $C_2$-$C_8$-alkylene, $C_4$-$C_{18}$-alkylene which is interrupted one or more times by -O-, 1,3- or 1-4-cyclohexylene or 1,3- or 1,4-phenylene,

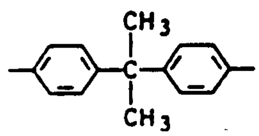 or 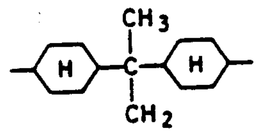

**5.** A compound as claimed in claim 1, wherein

$R^1$ and $R^2$ are each independently of the other H, Cl, methyl, ethyl, methoxy, ethoxy, benzyl, methylbenzyl or $\alpha,\alpha$-dimethylbenzyl,

$R^3$ and $R^4$ are each independently of the other H, $C_1$-$C_6$-alkyl, $C_1$-$C_8$-alkoxy, $C_7$-$C_9$-phenylalkyl, $C_7$-$C_9$-phenylalkoxy, phenoxy, benzoyloxy, $C_1$-$C_6$-alkanoyloxy, $C_1$-$C_6$-alkanoylamino or benzoylamino or $R^3$ and $R^4$ together are methylenedioxy or ethylenedioxy,

X is $C_1$-$C_5$-alkylene,

n is 1 and

$R^5$ is $-OR^6$ or

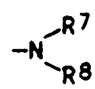

where

$R^6$ is H, $C_1$-$C_{18}$-alkyl, $C_3$-$C_{18}$-alkyl which is interrupted one or more times by -O- and which may in addition be -OH-substituted, unsubstituted or OH-substituted $C_5$-$C_{12}$-cycloalkyl, $C_2$-$C_{18}$-alkenyl, $C_7$-$C_9$-phenylalkyl, phenoxyethyl,

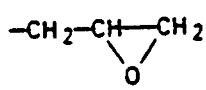 or 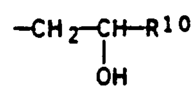 ,

one of the radicals

$R^7$ and $R^8$ is H and the other is $C_1$-$C_{12}$-alkyl, $C_3$-$C_{18}$-alkyl which is interrupted one or more times by -O- or $-NR^9-$, $C_5$-$C_{12}$-cycloalkyl, phenyl, $C_9$-phenylalkyl or $C_2$-$C_4$-hydroxyalkyl,

$R^9$ is H or $C_1$-$C_4$-alkyl,

$R^{10}$ is H, $C_1$-$C_{12}$-alkyl or $-CH_2OR^{11}$ and

$R^{11}$ is $C_1$-$C_{12}$-alkyl, $C_3$-$C_4$-alkenyl, cyclohexyl, phenyl or $C_7$-$C_9$-phenylalkyl.

**6.** A compound as claimed in claim 1, wherein

$R^1$ and $R^2$ are each independently of the other H, Cl, methyl, ethyl, methoxy, ethoxy, benzyl, methylbenzyl or $\alpha,\alpha$-dimethylbenzyl,

$R^3$ and $R^4$ are each independently of the other H, $C_1$-$C_6$-alkyl, $C_1$-$C_8$-alkoxy, $C_7$-$C_9$-phenylalkyl, $C_7$-$C_9$-phenylalkoxy, phenoxy, benzoyloxy, $C_1$-$C_6$-alkanoyloxy, $C_1$-$C_6$-alkanoylamino or benzoylamino or $R^3$ and $R^4$ together are methylenedioxy or ethylenedioxy,

X is $C_1$-$C_5$-alkylene,

n is 2 and
$R^5$ is a divalent radical of the formula
$-O-R^{12}-O-$ or

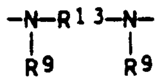

where
$R^9$ is H, $C_1$-$C_6$-alkyl or cyclohexyl,
$R^{12}$ is $C_2$-$C_6$-alkylene or $C_4$-$C_{18}$-alkylene which is interrupted one or more times by -O-, and
$R^{13}$ is $C_2$-$C_{12}$-alkylene which may be interrupted one or more times by -O-, or cyclohexylene.

**7.** The use of the compound as claimed in claim 1, 2, 3, 4, 5 or 6 as a light stabilizer for organic materials.

**8.** A stabilized organic material containing from 0.05 to 10 % by weight, based on the organic material, of one or more compounds as claimed in claim 1, 2, 3, 4, 5 or 6.

**9.** A stabilized organic material as claimed in claim 8, wherein the material is a synthetic polymer.

**10.** A stabilized organic material as claimed in claim 9, wherein the polymer is a polyester, polyamide, a hot-crosslinkable acrylic resin, a thermoplastic acrylic resin, a melamine-formaldehyde or urea-formaldehyde resin, ABS or a polyurethane.

**11.** A surface coating containing a stabilized organic material as claimed in claim 10.

## Revendications

**1.** Composés de formule I

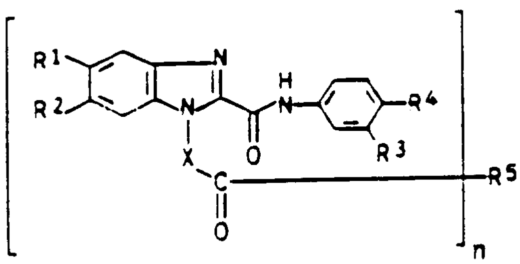

(I),

dans laquelle
$R^1$ et $R^2$ dont mis chacun, indépendamment l'un de l'autre, pour H, pour Cl, pour un reste alkyle en $C_1$-$C_4$, pour un reste alcoxy en $C_1$-$C_4$, pour un reste phényle éventuellement substitué par un radical alkyle en $C_1$-$C_4$ ou alcoxy en $C_1$-$C_4$ ou pour un reste phénalkyle en $C_7$-$C_9$,
$R^3$ et $R^4$ sont mis chacun, indépendamment l'un de l'autre, pour H, pour un reste alkyle en $C_1$-$C_{18}$, pour un reste alkyle en $C_3$-$C_{18}$ interrompu une ou plusieurs fois par -O-, pour un reste alcoxy en $C_1$-$C_{18}$, pour un reste alcoxy en $C_4$-$C_{18}$ interrompu une ou plusieurs fois par -O-, pour un reste phényle éventuellement substitué par un radical alkyle en $C_1$-$C_4$ ou alcoxy en $C_1$-$C_4$, pour un reste phénylalkyle en $C_7$-$C_{15}$, pour un reste phénoxy, pour un reste phénylalkyloxy en $C_7$-$C_{15}$,pour un reste hydroxyalkyle en $C_1$-$C_4$, pour un reste hydroxyalcoxy en $C_1$-$C_4$, pour un reste alkylcarbonylamino en $C_1$-$C_{12}$, pour un reste alcanoyloxy en $C_1$-$C_{12}$, pour un reste benzoylamino ou pour un reste benzoyloxy, ou $R^3$ et $R^4$ forment ensemble un reste méthylènedioxy ou éthylènedioxy,

X est mis pour un reste alkylène en $C_1$-$C_5$,

n est mis pour 1 ou 2 et

$R^5$

a) est mis - lorsque n = 1 - pour Cl, -$OR^6$ ou

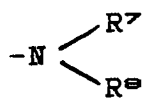

ou

b) est mis - lorsque n = 2 - pour un reste bivalent de formule -0-$R^{12}$-O- ou

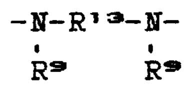

où

$R^6$ représente H, un reste alkyle en $C_1$-$C_{18}$ substitué par 1 à 3 groupements OH ou par -O-$COR^9$, un reste alkyle en $C_3$-$C_{18}$ qui est interrompu une ou plusieurs fois par -O- ou -$NR^9$- et qui est éventuellement substitué par -OH, un reste cycloalkyle en $C_5$-$C_{12}$ éventuellement substitué par OH, un reste alcényle en $C_2$-$C_{18}$ éventuellement substitué par OH, un reste phénylalkyle en $C_7$-$C_{15}$, un reste phénoxyéthyle ou un groupement

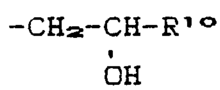 ou 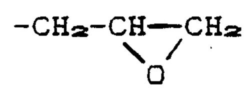

$R^7$ et $R^8$ représentent chacun, indépendamment l'un de l'autre, H, un reste alkyle en $C_1$-$C_{18}$, un reste alkyle en $C_3$-$C_{18}$ interrompu une ou plusieurs fois par -O- ou -$NR^9$-, un reste cycloalkyle en $C_5$-$C_{12}$, un reste phényle, un reste naphtyle, un reste alcényle en $C_3$-$C_{18}$, un reste phénylalkyle en $C_7$-$C_{15}$, un reste hydroxyalkyle en $C_2$-$C_4$, un reste

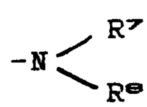

ou un reste pyrrolidinyle, pipéridinyle, pipérazinyle ou morpholinyle,

$R^9$ représente H, un reste alkyle en $C_1$-$C_{18}$, cycloalkyle en $C_5$-$C_{12}$, alcényle en $C_3$-$C_8$, décényle, oléyle, phényle, naphtyle ou phénylalkyle en $C_7$-$C_{18}$,

$R^{10}$ représente H, un reste alkyle en $C_1$-$C_{18}$, phénylalkyle en $C_7$-$C_{18}$, phényle éventuellement substitué ou -$CH_2OR^{11}$,

$R^{11}$ représente un reste alkyle en $C_1$-$C_{18}$, alcényle en $C_3$-$C_{18}$, cycloalkyle en $C_5$-$C_{12}$, phényle ou phénylalkyle en $C_7$-$C_{15}$,

$R^{12}$ représente un reste alkylène en $C_2$-$C_8$, alcénylène en $C_4$-$C_8$, cyclohexylène, alkylène en $C_4$-$C_{18}$ interrompu une ou plusieurs fois par -O-,

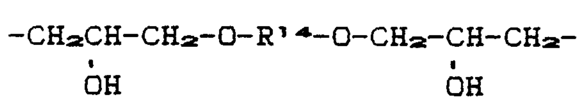 ou 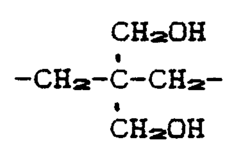

$R^{13}$ représente un reste alkylène en $C_2$-$C_{12}$ éventuellement interrompu une ou plusieurs fois par -O-, cyclohexylène,

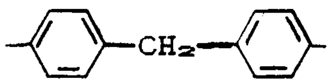 ou 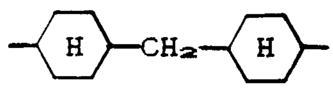 ou

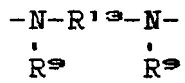

représente un reste pipérazine bivalent, et

$R^{14}$ représente un reste alkylène en $C_2$-$C_8$, alkylène en $C_4$-$C_{18}$ interrompu une ou plusieurs fois par -O-, 1,3- ou 1,4-cyclohexylène, 1,3- ou 1,4-phénylène,

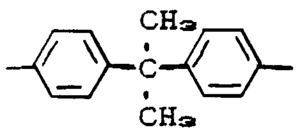 ou 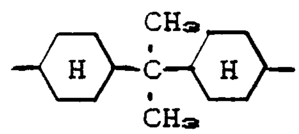 .

**2.** Composés selon la revendication 1, caractérisés en ce que

$R^1$ et $R^2$ sont mis chacun, indépendamment l'un de l'autre, pour H, Cl, un reste alkyle en $C_1$-$C_4$, alcoxy en $C_1$-$C_4$ ou phénylalkyle en $C_7$-$C_9$,

$R^3$ et $R^4$ sont mis chacun, indépendamment l'un de l'autre, pour H, pour un reste alkyle en $C_1$-$C_{12}$, pour un reste alcoxy en $C_1$-$C_{12}$, pour un reste alkyle en $C_3$-$C_{10}$ ou alcoxy en $C_3$-$C_{10}$ interrompu une ou plusieurs fois par -O-, pour un reste phénylalkyle en $C_7$-$C_9$, phénylalcoxy en $C_7$-$C_9$, phénoxy, alkylcarbonyloxy en $C_1$-$C_{12}$, benzoyloxy, alkylcarbonylamino en $C_1$-$C_{12}$, benzoylamino, ou $R^3$ et $R^4$ forment ensemble un reste méthylènedioxy ou éthylènedioxy, et

X est mis pour un reste alkylène en $C_1$-$C_5$.

**3.** Composés selon la revendication 2, caractérisés en ce que

n est mis pour 1 et

$R^5$ est mis pour $-OR^6$ ou

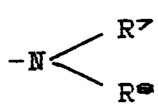

où

$R^6$ représente H, un reste alkyle en $C_1$-$C_{18}$ qui est éventuellement substitué par -OH ou $-OCOR^9$, un reste alkyle en $C_3$-$C_{18}$ qui est interrompu une ou plusieurs fois par -O- et qui est éventuellement substitué par -OH, un reste cycloalkyle en $C_5$-$C_{12}$ ou alcényle en $C_2$-$C_{18}$ éventuellement substitué par -OH, un reste phénylalkyle en $C_7$-$C_{15}$, phénoxyéthyle,

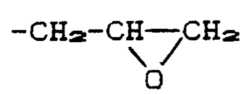

ou 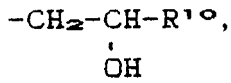

$R^7$ et $R^8$ sont mis chacun, indépendamment l'un de l'autre, pour H, pour un reste alkyle en $C_1$-$C_{12}$, pour un reste alkyle en $C_3$-$C_{18}$ interrompu une ou plusieurs fois par -O- ou -$NR^9$-, pour un reste cycloalkyle en $C_5$-$C_{12}$, phényle, phénylalkyle en $C_7$-$C_{15}$ ou hydroxyalkyle en $C_2$-$C_4$ ou pour un reste

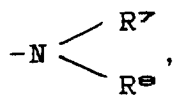

pyrrolidine, pipéridine, pipérazine ou morpholine,

$R^9$ représente H, un reste alkyle en $C_1$-$C_4$, cycloalkyle en $C_5$-$C_{12}$, phényle ou benzyle,

$R^{10}$ représente H, un reste alkyle en $C_1$-$C_{12}$ ou -$CH_2OR^{11}$

et

$R^{11}$ représente un reste alkyle en $C_1$-$C_{12}$, cycloalkyle en $C_5$-$C_{10}$, phényle ou phénylalkyle en $C_7$-$C_9$.

**4.** Composés selon la revendication 2, caractérisés en ce que

n est mis pour 2 et

$R^5$ est mis pour un reste bivalent de formule -O-$R^{12}$-O- ou

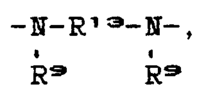

où

$R^9$ représente H, un reste alkyle en $C_1$-$C_{18}$ ou cyclohexyle,

$R^{12}$ représente un reste alkylène en $C_2$-$C_8$, alkylène en $C_4$-$C_{18}$ interrompu une ou plusieurs fois par -O- ou

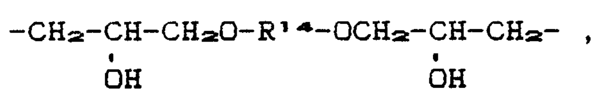

$R^{13}$ représente un reste alkylène en $C_2$-$C_{12}$ éventuellement interrompu une ou deux fois par -O-, cyclohexylène,

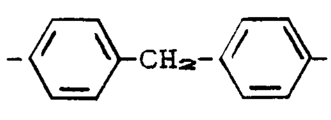

ou

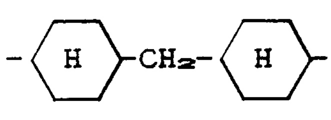

et

$R^{14}$ représente un reste alkylène en $C_2$-$C_8$, alkylène en $C_4$-$C_{18}$ interrompu une ou plusieurs fois par -O-, 1,3- ou 1,4-cyclohexylène, 1,3- ou 1,4-phénylène,

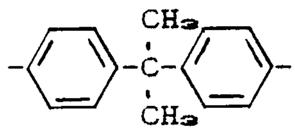 ou 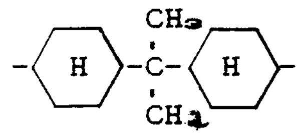 .

**5.** Composés selon la revendication 1, caractérisés en ce que

$R^1$ et $R^2$ sont mis chacun, indépendamment l'un de l'autre, pour H, Cl, un reste méthyle, éthyle, méthoxy, éthoxy, benzyle, méthylbenzyle ou α,α-diméthylbenzyle,

$R^3$ et $R^4$ sont mis chacun, indépendamment l'un de l'autre, pour H, un reste alkyle en $C_1$-$C_6$, alcoxy en $C_1$-$C_8$, phénylalkyle en $C_7$-$C_9$, phénylalcoxy en $C_7$-$C_9$, phénoxy, benzoyloxy, alcanoyloxy en $C_1$-$C_6$, alcanoylamino en $C_1$-$C_6$ ou benzoylamino, ou $R^3$ et $R^4$ sont mis ensemble pour un reste méthylènedioxy ou éthylènedioxy,

X est mis pour un reste alkylène en $C_1$-$C_6$,

n est mis pour 1 et

$R^5$ est mis pour $-OR^6$ ou

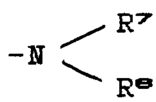 ,

où

$R^6$ représente H, un reste alkyle en $C_1$-$C_{18}$, alkyle en $C_3$-$C_{18}$ qui est interrompu une ou plusieurs fois par -O- et qui est éventuellement substitué par -OH, cycloalkyle en $C_5$-$C_{12}$ éventuellement substitué par -OH, alcényle en $C_2$-$C_{18}$, phénylalkyle en $C_7$-$C_9$, phénoxyéthyle,

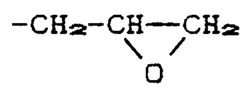 ou 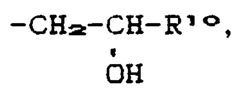 ,

l'un des restes $R^7$ et $R^8$ représente H et l'autre des restes $R^7$ et $R^8$ est un reste alkyle en $C_1$-$C_{12}$, alkyle en $C_3$-$C_{18}$ interrompu une ou plusieurs fois par -O- ou $-NR^9$-, cycloalkyle en $C_5$-$C_{12}$, phényle, phénylalkyle en $C_9$ ou hydroxyalkyle en $C_2$-$C_4$,

$R^9$ représente H ou un reste alkyle en $C_1$-$C_4$,

$R^{10}$ représente H, un reste alkyle en $C_1$-$C_{12}$ ou $-CH_2OR^{11}$ et

$R^{11}$ représente un reste alkyle en $C_1$-$C_{12}$, alcényle en $C_3$-$C_4$, cyclohexyle, phényle ou phénylalkyle en $C_7$-$C_9$.

**6.** Composés selon la revendication 1, caractérisés en ce que

$R^1$ et $R^2$ sont mis chacun, indépendamment l'un de l'autre, pour H, Cl, un reste méthyle, éthyle, méthoxy, éthoxy, benzyle, méthylbenzyle ou α,α-diméthylbenzyle,

$R^3$ et $R^4$ sont mis chacun, indépendamment l'un de l'autre, pour H, un reste alkyle en $C_1$-$C_6$, alcoxy en $C_1$-$C_8$, phénylalkyle en $C_7$-$C_9$, phénylalcoxy en $C_7$-$C_9$, phénoxy, benzoyloxy, alcanoyloxy en $C_1$-$C_6$, alcanoylamino en $C_1$-$C_6$ ou benzoylamino, ou $R^3$ et $R^4$ forment ensemble un reste méthylènedioxy ou éthylènedioxy,

X est mis pour un reste alkylène en $C_1$-$C_5$,

n est mis pour 2 et

$R^5$ est mis pour un reste bivalent de formule

$-O-R^{12}-O-$ ou

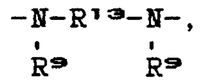

où

$R^9$ représente H, un reste alkyle en $C_1$-$C_6$ ou cyclohexyle,

$R^{12}$ représente un reste alkylène en $C_2$-$C_6$ ou alkylène en $C_4$-$C_{18}$ interrompu une ou plusieurs fois par -O-, et

$R^{13}$ représente un reste alkylène en $C_2$-$C_{12}$ éventuellement interrompu une ou plusieurs fois par -O- ou un reste cyclohexylène.

**7.** Utilisation des composés selon l'une quelconque des revendications 1 à 6 comme agent de protection contre la lumière pour des matières organiques.

**8.** Matière organique stabilisée, contenant de 0,05 à 10% en poids - par rapport à la matière organique - d'un ou de plusieurs composés selon l'une quelconque des revendications 1 à 6.

**9.** Matière organique stabilisée selon la revendication 8, caractérisée en ce que la matière est un polymère synthétique.

**10.** Matière organique stabilisée selon la revendication 9, caractérisée en ce que le polymère est un polyester, un polyamide, une résine acrylique thermoréticulable, une résine acrylique thermoplastique, une résine MF ou UF, un ABS ou un polyuréthanne.

**11.** Vernis, contenant une matière organique stabilisée selon la revendication 10.